# EUROPEAN PATENT APPLICATION

(11) **EP 1 836 945 A2**
(43) Date of publication of application: **26.09.2007**
(21) Application number: 07004684.2
(22) Date of filing: 07.03.2007
(51) Int. Cl.: A61B 1/00

(54) **Switch mechanism for use in medical apparatuses, and image-pickup device for use in endoscopes**

(30) Priority: 20.03.2006 JP 2006077090; 20.03.2006 JP 2006077091
(71) Applicant: OLYMPUS MEDICAL SYSTEMS CORP., Shibuya-ku Tokyo 151-0072 (JP)
(72) Inventor: Shimizu, Masami, c/o Int. Prop. Support Dept., Hachioji-shi Tokyo 192-8512 (JP); Takekoshi, Satoshi, c/o Int. Prop. Support Dept., Hachioji-shi Tokyo 192-8512 (JP); Takayama, Masaki, c/o Int. Prop. Support Dept., Hachioji-shi Tokyo 192-8512 (JP); Hagihara, Masahiro, c/o Int. Prop. Support Dept., Hachioji-shi Tokyo 192-8512 (JP); Omachi, Kenji, c/o Int. Prop. Support Dept., Hachioji-shi Tokyo 192-8512 (JP); Yamaguchi, Takao, c/o Int. Prop. Support Dept., Hachioji-shi Tokyo 192-8512 (JP)
(74) Representative: von Hellfeld, Axel

(57) **Abstract**

A switch mechanism (52) for use in medical apparatuses, includes an airtight unit (46) being able to retain airtight therein, a first magnet (114) provided outside the airtight unit, an operation unit (112) provided outside the airtight unit and being able to move the first magnet with respect to the airtight unit, a second magnet (128) provided in the airtight unit and being able to receive a force from the first magnet in accordance with a position of the first magnet, and a switch unit (122) provided in the airtight unit and being able to be switched on and off by using a force acting between the first magnet and the second magnet.

## Description

The present invention relates to a switch mechanism for use in various types of medical apparatuses and an image-pickup device for use in endoscopes.

In recent years, autoclave sterilization using high-temperature, high-pressure steam has been widely used as a method of sterilizing endoscopes and image-pickup devices. Electronic endoscopes including a thin and long insertion section to be inserted into a body cavity, the insertion section being inserted into the body cavity to observe and perform treatment in the cavity, or Image-pickup devices for use in rigid scopes, each to be attached to the eyepiece section of the rigid scopes, taking endoscopic images, generally have a remote switch that is operated to adjust the brightness of endoscopic images, to freeze the images, to magnify the endoscopic images and to adjust the focal distance.

An example of such an endoscope that has a remote switch and can be sterilized by the autoclave sterilization is disclosed in, for example, Jpn. Pat. Appln. KOKAI Publication No. 2000-139819. The endoscope has an airtight unit into which steam would not flow during the autoclave sterilization. The optical lens provided in the airtight unit is moved as the remote switch provided outside the airtight unit is operated. Thus, the position of the focus is adjusted.

A switch mechanism for use in medical apparatuses, according to the present invention, includes:
an airtight unit which is able to retain airtight therein;
a first magnet which is provided outside the airtight unit;
an operation unit on which the first magnet is arranged, which is provided outside the airtight unit and which is able to move the first magnet with respect to the airtight unit;
a second magnet which is provided in the airtight unit and which is able to receive a force from the first magnet in accordance with a position of the first magnet; and
a switch unit which is provided in the airtight unit and which is able to be switched on and off by using a force acting between the first magnet and the second magnet.

Another switch mechanism for use in medical apparatuses, according to this, includes:
an airtight unit which is able to retain airtight therein;
a magnetic switch which is provided in the airtight unit and which is able to detect changes in a magnetic force;
a magnetic-force generating unit which is provided outside the airtight unit; and
a magnetic-force changing mechanism which is able to change a magnetic force acting between a magnet and the magnetic switch.

Still another switch mechanism for use in medical apparatuses, according to the present invention, includes:
an airtight unit which is able to retain airtight therein;
a first magnet which is provided outside the airtight unit;
an operation unit on which the first magnet is arranged, which is provided outside the airtight unit and which is able to move the first magnet with respect to the airtight unit;
a second magnet which is provided in the airtight unit and which is able to receive a force from the first magnet in accordance with a position of the first magnet; and
a detection switch unit which is provided in the airtight unit, which has a light-emitting part being able to emit light and a light-receiving part being able to receive light emitted from the light-emitting part, and which is able to detect a change in an amount of light received by the light-receiving part, in accordance with the position of the second magnet.

The invention can be more fully understood from the following detailed description when taken in conjunction with the accompanying drawings, in which:
FIG. 1 is a schematic diagram showing an endoscope system according to a first embodiment of the invention;
FIG. 2 is a longitudinal sectional view schematically showing the structure of the endoscope system according to the first embodiment of the invention;
FIGS. 3A and 3B are longitudinal sectional views showing the switch device provided in the image-pickup device used in the endoscope system according to the first embodiment of the invention;
FIGS. 4A to 4C are schematic diagrams, each showing the tactile switch and a modified second magnet, both used in the switch device provide on the image-pickup device used in the endoscope system according to the first embodiment of the invention;
FIGS. 5A and 5B are longitudinal sectional views schematically showing the switch device provided in the image-pickup device used in the endoscope system according to a second embodiment of the invention;
FIGS. 6A and 6B are longitudinal sectional views schematically showing the switch device provided in the image-pickup device used in the endoscope system according to a third embodiment of the invention;
FIGS. 7A and 7B are longitudinal sectional views schematically showing a modification of the switch device provided in the image-pickup device used in the endoscope system according to the third embodiment of the invention;
FIGS. 8A and 8B are longitudinal sectional views schematically showing the switch device provided in the image-pickup device used in the endoscope system according to a fourth embodiment of the invention;
FIGS. 9A and 9B are longitudinal sectional views schematically showing a modification of the switch device provided in the image-pickup device used in the endoscope systems according to the first to fourth embodiments of the invention;
FIGS. 10A and 10B are longitudinal sectional views schematically showing another modification of the switch device provided in the image-pickup device used in the endoscope systems according to the first to fourth embodiments of the invention;
FIGS. 11A and 11B are longitudinal sectional views schematically showing still another modification of the switch device provided in the image-pickup device used in the endoscope systems according to the first to fourth embodiments of the invention;
FIG. 12 is a longitudinal sectional view schematically showing the switch device provided in the image-pickup device used in the endoscope system according to a fifth embodiment of the invention;
FIGS. 13A to 13C are plan views, taken along line XIII-XIII in FIG. 12, schematically showing the switch device provided in the image-pickup device used in the endoscope system according to the fifth embodiment of the invention;
FIGS. 14A to 14C are plan views, taken along line XIV-XIV in FIG. 12, schematically showing the switch device provided in the image-pickup device used in the endoscope system according to the fifth embodiment of the invention;
FIG. 15A is a longitudinal sectional view showing the switch device provided in the image-pickup device used in the endoscope system according to a sixth embodiment of the invention;
FIG. 15B is a perspective view schematically showing the light-cutoff member provided in the switch provided in the image-pickup device used in the endoscope system according to the sixth embodiment of the invention;
FIG. 16 is a perspective view schematically showing the optical switch and the light-cutoff member, both provided in the switch provided in the image-pickup device used in the endoscope system according to a seventh embodiment of the invention;
FIG. 17 is a longitudinal sectional view showing the switch device provided in the image-pickup device used in the endoscope system according to an eighth embodiment of the invention;
FIG. 18 is a longitudinal sectional view showing the switch device provided in the image-pickup device used in the endoscope system according to a ninth embodiment of the invention;
FIG. 19 is a longitudinal sectional view showing the switch device provided in the image-pickup device used in the endoscope system according to a tenth embodiment of the invention;
FIG. 20 is a schematic diagram showing the outer appearance of the switch device provided in the image-pickup device used in the endoscope system according to an eleventh embodiment of the invention;
FIGS. 21A and 21B are longitudinal sectional views schematically showing the switch device provided in the image-pickup device used in the endoscope system according to the eleventh embodiment of the invention;
FIG. 22 is a longitudinal sectional view schematically showing a modification of the slide switch, which may be provided in the image-pickup device used in the endoscope system according to the eleventh embodiment of the invention;
FIGS. 23A and 23B are longitudinal sectional views schematically showing the switch device provided in the image-pickup device used in the endoscope system according to a twelfth embodiment of the invention;
FIGS. 24A and 24B are longitudinal sectional views schematically showing the switch device provided in the image-pickup device used in the endoscope system according to a thirteenth embodiment of the invention;
FIG. 25 is a longitudinal sectional view schematically showing the switch device provided in the image-pickup device used in the endoscope system according to a fourteenth embodiment of the invention;
FIG. 26 is a longitudinal sectional view schematically showing the switch device provided in the image-pickup device used in the endoscope system according to a fifteenth embodiment of the invention;
FIG. 27 is an exploded perspective view schematically showing the switch device provided in the image-pickup device used in the endoscope system according to a sixteenth embodiment of the invention;
FIG. 28 is a longitudinal sectional view schematically showing the switch device that may be provided in the image-pickup device used in the endoscope system according to the sixteenth embodiment of the invention;
FIG. 29 is a longitudinal sectional view schematically showing the switch device provided in the image-pickup device used in the endoscope system according to a seventeenth embodiment of the invention;
FIGS. 30A and 308 are longitudinal sectional views schematically showing the switch device provided in the image-pickup device used in the endoscope system according to an eighteenth embodiment of the invention;
FIG. 31 is a perspective view schematically showing a modification of the switch device provided in the image-pickup device used in the endoscope system according to the eighteenth embodiment of the invention;
FIG. 32 is a longitudinal sectional view schematically showing a modification of the switch device provided in the image-pickup device used in the endoscope system according to the eighteenth embodiment of the invention;
FIGS. 33A and 338 are longitudinal sectional views schematically showing the switch device provided in the image-pickup device used in the endoscope system according to a nineteenth embodiment of the invention;
FIG. 34 is a longitudinal sectional view schematically showing the structure of an image-pickup device for use in the endoscope system according to a twentieth embodiment of the invention;
FIGS. 35A and 35B are longitudinal sectional views schematically showing the switch device provided in the image-pickup device used in the endoscope system according to the twentieth embodiment of the invention;
FIGS. 36A and 36B are longitudinal sectional views schematically showing the switch device provided in an image-pickup device used in the endoscope system according to a twenty-first embodiment of the invention;
FIGS. 37A and 37B are longitudinal sectional views schematically showing the switch device provided in the image-pickup device used in the endoscope system according to a twenty-second embodiment of the invention;
FIGS. 38A and 38B are longitudinal sectional views schematically showing the switch device provided in the image-pickup device used in the endoscope system according to a twenty-third embodiment of the invention;
FIGS. 39A and 39B are longitudinal sectional views schematically showing the switch device provided in the image-pickup device used in the endoscope system according to a twenty-fourth embodiment of the invention;
FIGS. 40A and 40B are longitudinal sectional views schematically showing the switch device provided in the image-pickup device used in the endoscope system according to a twenty-fifth embodiment of the invention;
FIG. 41 is a longitudinal sectional view schematically showing the structure of the endoscope system according to a twenty-sixth embodiment of the invention;
FIG. 42 is a schematic diagram showing the arrangement of magnets in the switch device provided in the image pickup device used in the endoscope system according to a twenty-seventh embodiment of the invention;
FIG. 43 is a schematic diagram showing the arrangement of magnets in the switch device provided in the image pickup device used in the endoscope system according to a twenty-eighth embodiment of the invention;
FIG. 44 is a longitudinal sectional view schematically showing the structure of the endoscope system according to a twenty-ninth embodiment of the invention;
FIG. 45 is a transverse sectional view schematically showing the switch device provided in the image-pickup device used in the endoscope system according to the twenty-ninth embodiment of the invention;
FIG. 46 is a longitudinal sectional view schematically showing the switch device provided in the image-pickup device used in the endoscope system according to a thirtieth embodiment of the invention;
FIG. 47 is a longitudinal sectional view schematically showing the switch device provided in the image-pickup device of the endoscope system according to a thirty-first embodiment of the invention;
FIG. 48 is a schematic longitudinal sectional view showing the second heat conductor of the switch device that is provided in the image-pickup device used in the endoscope system according to a thirty-second embodiment of the invention;
FIGS. 49A and 49B are schematic longitudinal sectional views showing the switch mechanism of the switch device that is provided in the image-pickup device used in the endoscope system according to a thirty-third embodiment of the invention;
FIGS. 50A and 50B are schematic longitudinal sectional views showing the switch mechanism of the switch device that is provided in the image-pickup device used in the endoscope system according to a thirty-fourth embodiment of the invention;
FIG. 51A is a schematic longitudinal sectional view showing the positional relation between the airtight frame, outer shell, magnetic sensor and operation member of the switch device that is provided in the image-pickup device used in the endoscope system according to a thirty-fifth embodiment of the invention;
FIG. 51B is a schematic perspective view showing the operation member used in the switch device that is provided in the image-pickup device used in the endoscope system according to the thirty-fifth embodiment of the invention;
FIG. 51C is a schematic diagram showing the operation member put on the operator's finger, said member used with the switch device provided in the image-pickup device used in the endoscope system according to the thirty-fifth embodiment of the invention;
FIG. 52 is a schematic perspective view of an operation member used with the switch device provided in the image-pickup device used in the endoscope system according to a thirty-sixth embodiment of the invention;
FIG. 53 is a schematic diagram showing the magnetic unit provided in the image-pickup device used in the endoscope system according to a thirty-seventh embodiment of the invention;
FIGS. 54A and 54B are longitudinal sectional views schematically showing the magnetic unit provided in the image-pickup device used in the endoscope system according to the thirty-seventh embodiment of the invention;
FIG. 55 is a longitudinal sectional view schematically showing the means for aligning the permanent magnets provided in the magnetic unit of the image-pickup device used in the endoscope system according to the thirty-seventh embodiment of the invention;
FIG. 56 is a schematic diagram showing the magnetic unit provided in the image-pickup device used in the endoscope system according to a thirty-eighth embodiment of the invention;
FIG. 57 is a schematic diagram showing a modification of magnetic unit provided in the image-pickup device used in the endoscope system according to the thirty-eighth embodiment of the invention;
FIG. 58 is a schematic diagram showing another modification of magnetic unit provided in the image-pickup device used in the endoscope system according to the thirty-eighth embodiment of the invention; and
FIG. 59 is a schematic diagram showing still another modification of magnetic unit provided in the image-pickup device used in the endoscope system according to the thirty-eighth embodiment of the invention.

The best mode for carrying out the present invention will be explained, with reference to the accompanying drawings.

### [First Embodiment]

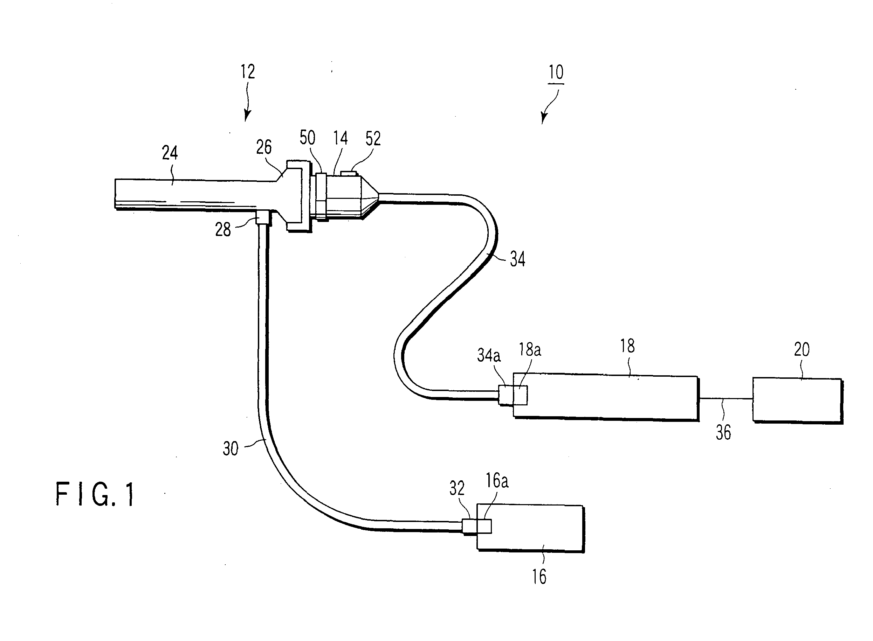

The first embodiment will be described with reference to FIGS. 1 to 4C.

### (Configuration)

As FIG. 1 shows, an endoscope system 10 according to the embodiment includes an endoscope 12, an image-pickup device for use in endoscopes (medical apparatuses) 14, a light source device 16, a video processor 18, and a monitor 20.

The endoscope 12 is used when inserted into a body cavity. The image-pickup device 14 is removably attached to the endoscope 12. The image-pickup device 14 can therefore photographs images through the endoscope 12. The light source device 16 is removably attached to the endoscope 12 to apply illumination light to the endoscope 12. The video processor 18 is removably connected to the image-pickup device 14 and can process signals transmitted from the image-pickup device 14. The monitor 20 is removably connected to the video processor 18 and can display images represented by video signals output from the video processor 18.

The endoscope 12 includes an insertion section 24, an eyepiece section 26, and a mouth ring 28. The eyepiece section 26 is provided at the proximal end of the insertion section 24. The mouth ring 28 is provided the side of the insertion section 24. A light-guiding cable 30 is connected at one end to the mouth ring 28. To the other end of the light-guiding cable 30, a connector 32 is connected. The light source device 16 has a receptacle 16a. The connector 32 can be attached to, and detached from, the receptacle 16a.

The insertion section 24 of the endoscope 12 contains an illumination window, an objective lens, a light guide, a relay optical system, and an ocular, which are not shown. The illumination window and the objective lens are arranged at the distal end of the insertion section 12. The ocular is arranged at the proximal end of the insertion section 24.

The illumination window, the light guide and the mouth ring 28 are optically connected. Since the light-guiding cable 30 is connected to the mouth ring 28, the illumination window and the light guide are optically connected to the light-guiding cable 30. Hence, daylight emitted from a lamp (not shown) provided in the light source device 16 is applied to the end of the light-guiding cable 30 once the connector 32 has connected the light-guiding cable 30 to the receptacle 16a of the light source device 16. Thus, illumination light is applied through the light guide provided in the insertion section 24 of the endoscope 12 and is emitted from the illumination window to the object of observation. The object is thereby illuminated.

The objective lens, the relay optical system and the ocular are optically connected. The light reflected from the object illuminated with the illumination light is applied to the objective lens, which forms an image of the object. The image thus formed (hereinafter mainly called endoscopic image) is conveyed from the objective lens to a relay lens. The image is conveyed from the relay lens to the ocular. Thus, the operator can observe the endoscopic image focused at the distal end of the insertion section 24, at the eyepiece section 26 that is provided in the proximal end of the insertion section 24.

The image-pickup device 14 is connected to the eyepiece section 26. The image-pickup device 14 therefore performs photoelectric conversion on the endoscopic image. The image is thereby converted into an electric signal.

As shown in FIG. 1, a camera cable 34 extends from the image-pickup device 14. A plug 34a is provided at the distal end of the camera cable 34. The video processor 18 has a receptacle 18a. The plug 34a is removably attached to the receptacle 18a. Therefore, the image-pickup device 14 and the video processor 18 are electrically connected to each other by the camera cable 34 and can be disconnected from each other. Note that the video processor 18 and the monitor 20 are electrically connected by a cable 36.

As shown in FIG. 1, too, the electric signal representing the endoscopic image formed by the image-pickup device 14 is supplied to the video processor 18 via the camera table 34 extending from the image-pickup device 14 and the plug 34a. The video processor 18 converts the electric signal to a video signal. The video signal is output to the monitor 20. The monitor 20 displays the endoscopic image.

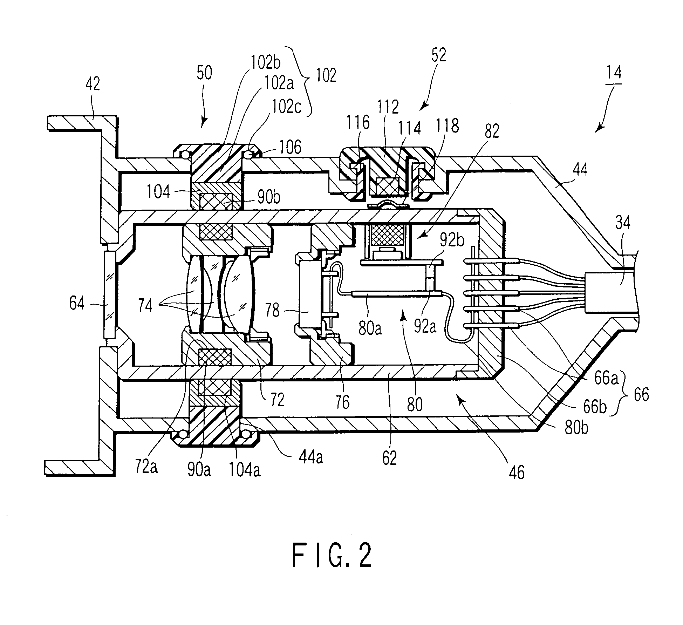

As indicated above, the image-pickup device 14 is removably attached to the eyepiece section 26 of the endoscope 12. The configuration of the image-pickup device (medical apparatuses) 14 for use in endoscopes will be described more specifically, with reference to FIGS. 2 to 3B.

As FIG. 2 shows, the image-pickup device 14 includes a scope mount 42, an outer shell 44, an airtight unit 46, a focus-adjusting unit 50, and a switch device 52. The focus-adjusting unit 50 and the switch device 52 are provided on the outer shell 44 and in the airtight unit 46.

The outer shell 44 is shaped like a hollow cylinder. The scope mount 42 is integrally formed with the distal end of the outer shell 44. The airtight unit 46 is provided in the outer shell 44. The focus-adjusting unit 50 and the switch device 52 are arranged in the outer shell 44.

The airtight unit 46 includes an airtight-unit body 62, cover glass 64, and a hermetic connector 66. The airtight-unit body 62 is shaped like a hollow cylinder. The airtight-unit body 62 is made by cutting a stainless steel block or a titanium block as in most cases. Instead, it may be produced by electro-casting. In this case, the body 62 can be smaller and lighter.

The airtight-unit body 62 is secured at one end to the outer shell 44. To this end of the airtight-unit body 62, the cover glass 64 is coupled in airtight fashion, by means of soldering (not shown) or the like. To the other end of the airtight-unit body 62, the hermetic connector 66 is coupled in airtight fashion, by means of high-frequency soldering, laser welding or the like. The hermetic connector 66 has a connecting pin 66a and a hermetic-connector body 66b. The pin 66a and the body 66b are insulated by glass sealing or a similar technique and are coupled to each other in airtight fashion. The airtight unit 46 retains airtight inside.

In the airtight unit 46 there are provided a lens fame 72, an optical system 74, a solid-state imaging element frame 76, a solid-state imaging element 78, a signal-transmitting circuit 80, and a switch unit 82. The switch unit 82 is a component of the switch device 52.

The lens frame 72 is arranged in the distal end of the airtight-unit body 62. The optical system 74 is secured in the center of the lens frame 72. Therefore, the optical system 74 is optically connected to the proximal end of the cover glass 64. The lens frame 72 can move along the optical axis of the optical system 74, while contacting the inner circumferential surface of the airtight-unit body 62. Thus, the optical system 74 receives the endoscopic image coming from the eyepiece section 26 of the endoscope 12 (shown in FIG. 1) through the cover glass 64.

A plurality of recesses 72a is made in the outer circumferential surface of the lens frame 72. The recesses 72a need not be connected to form an annular recess. They only need to be spaced from one another. It is desired that the recesses 72a be spaced at regular intervals around the axis of the lens frame 72. First inner magnets (magnets on the lens frame) 90a are fitted in the recesses 72a, respectively.

The signal-transmitting circuit 80 includes, for example, a flex-rigid board (first board) that is composed of a hard part 80a and a soft part 80b. On the hard part 80a, a first connector 92a is provided to transmit electric signals from the switch unit 82.

The focus-adjusting unit 50 includes a focus ring 102, a movable part 104, first elastic members 106 such as O rings, and a first outer magnet (focusing magnet) 90b.

The outer shell 44 has an annular groove 44a that extends around its axis. In the groove 44a, the focus ring 102 is fitted. The outer edge of the focus ring 102 has a T-shaped cross section. That is, the focus ring 102 includes a ring part 102a and a flange part 102b that protrudes from the outer circumference of the ring part 102a along the axis of the outer shell 44. The focus ring 102 can therefore rotate, sliding in the annular groove 44a of the outer shell 44.

The flange part 102b of the focus ring 102 has a pair of recesses 102c cut in the side that faces the outer circumferential surface of the outer shell 44. The elastic members 106 are fitted in these recesses 102c, respectively. Thus, the junction between the focus ring 102 and the outer shell 44 retains watertight. Further, the friction between the first elastic members 106 and the focus ring 102 and the friction between the first elastic members 106 and the outer shell 44 prevent the focus ring 102 from making an unnecessarily rotation.

A rotation-angle restricting means (e.g., a cam mechanism composed of a cam pin and a cam groove, not shown) prevents the focus ring 102 from rotating through an angle greater than a predetermined angle. Therefore, the focus ring 102 can rotate through an angle within a preset range. The movable part 104 is shaped like a ring. The outer circumferential surface of the movable part 104 and the inner circumferential surface of the focus ring 102 are coupled by a cam mechanism or the like. Hence, the movable part 104 moves on the outer circumferential surface of the airtight-unit body 62 in the axial direction thereof when the focus ring 102 is rotated.

The movable part 104 has recesses 104a made in its inner circumferential surface. First outer magnets 90b are fitted in the recesses 104a and face the first inner magnets 90a, respectively, across the wall of the airtight-unit body 62.

The first outer magnets 90b are magnetically coupled with the first inner magnets 90a. Because of the magnetic coupling of the first outer magnets 90b and first inner magnets 90a, the lens frame 72 therefore moves along the optical axis when the movable part 104 moves along the optical axis. Hence, the optical system 74 moves, adjusting the position of the focus, when the focus ring 102 is rotated.

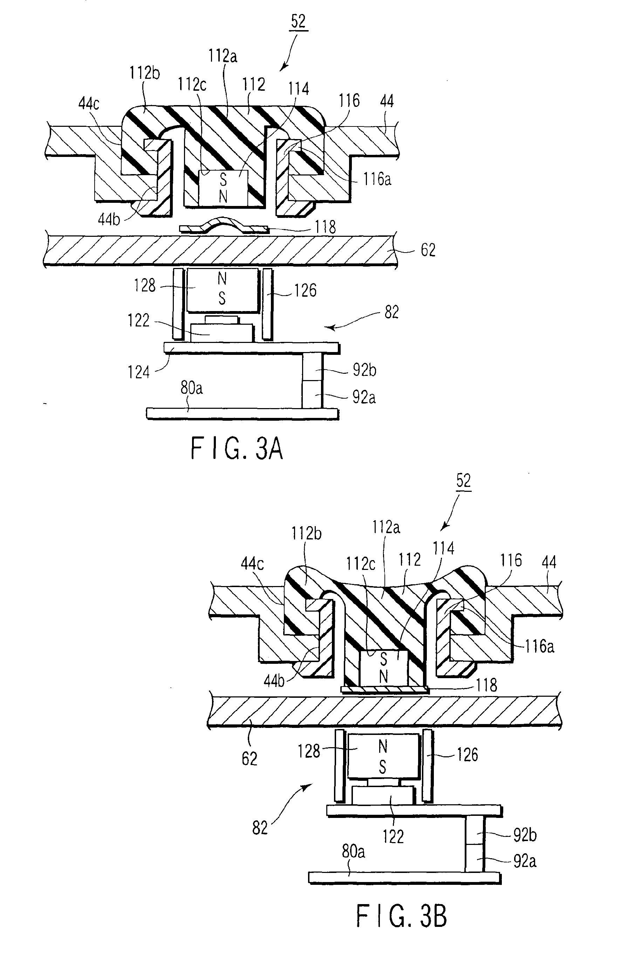

As shown in FIG. 3A, the switch device 52 includes the above-mentioned switch unit 82, an operation unit (magnetic-force changing mechanism) 112, a first magnet 114, a fastening member 116, and a leaf spring 118.

The operation unit 112 includes a base part (e.g., recessed part) 112a, a bending part (return mechanism) 112b, and a recess (magnet-holding part) 112c. The base part 112a is shaped like, for example, a column. The bending part 112b extends outwards in the radial direction from one end of the base part 112a. The recess 112c is made in the other end of the base part 112a.

The outer shell 44 has a through hole 44b that extends toward its axis. A recessed part 44c is provided around the through hole 44b and opens toward the axis of the outer shell 44. The operation unit 112 is fitted in the recessed part 44c and extends through the through hole 44b. The recessed part 44c and the operation unit 112 are held by the fastening member 116 that is shaped like a ring and has a flange part 116a. That is, the fastening member 116 secures the operation unit 112 to the outer shell 44, preventing the operation unit 112 from slipping from the outer shell 44.

The operation unit 112 is made of elastic material such as silicone rubber and is shaped like a button having a cross section that is shaped like letter T. Therefore, the bending part 112b is deformed when the operator pushes the operation unit 112 with a finger, but restores its initial shape when the operator stops pushing the operation unit 112. Thus, the operation unit 112 changes in position and shape, assuming the first position (see FIG. 3A) until it is pushed, and the second position (see FIG. 3B) when it is pushed. As indicated above, the operation unit 112 has the recess 112c. In the recess 112c, the first magnet 114 is fitted and integrally formed with the unit 112 by means of adhesion or molding. Note that the first magnet 114 has its N and S poles specifically positioned, for example, as illustrated in FIG. 3A.

The leaf spring 118 is provided on the outer circumferential surface of the airtight-unit body 62. The leaf spring 118 is deformed as it is pressed onto the first magnet 114 when the operation unit 112 is operated. As it is so deformed, the leaf spring 118 gives a sense of click to the operator who is operating the operation unit 112.

As shown in FIGS. 3A and 3B, the switch unit 82 includes a tactile switch (switch part) 122, a rigid board (second board) 124, a guide 126, and a second magnet 128.

The tactile switch 122 is mounted on one side of the rigid board 124. The "one side" of the rigid board 124 faces the airtight-unit body 62. The guide 126 is mounted on the one side of the rigid board 124, too, and surrounds the tactile switch 122. The second magnet 128 is provided in the guide 126. The second magnet 128 have its S and N poles specifically positioned, for example, as illustrated in FIG. 3A. Therefore, the first magnet 114 and the second magnet 128 apply repulsive force to each other when they are made to approach each other.

The tactile switch 122 and the second magnet 128 are arranged, opposed to each other. The tactile switch 122 contains an elastic member (not shown) such as a leaf spring (as in the embodiment). The tactile switch 122 is usually turned off, by virtue of the bias (spring force) of this leaf spring, in spite of the weight of the second magnet 128 exerted on the tactile switch 122.

On the other side of the rigid board 124, a second connector 92b is provided. The second connector 92b is electrically connected to the first connector 92a. Hence, the electric signal generated when the tactile switch 122 is turned on or off is transmitted to the hard part 80a via the rigid board 124, second connector 92b and first connector 92a.

### (Operation)

How the endoscope system 10 according to the embodiment operates will be explained, mainly as to how the switch device 52 operates.

Assume that the switch device 52 has the function of increasing the brightness of endoscopic images. Then, as shown in FIG. 1, the image-pickup device 14 is removably attached to the eyepiece section 26 that is provided at the proximal end of the insertion section 24 of the endoscope 12. The device 14 therefore serves to accomplish endoscopic examination.

Illumination light is applied from the light source device 16 to the insertion section 24 through the receptacle 16a, connector 32, light-guiding cable 30 and mouth ring 28. The light is emitted from the illumination lens (not shown) provided in the distal end of the insertion section 24. The light thus emitted illuminates the object. The image of the object illuminated, or an endoscopic image, is applied to the objective lens provided in the distal end of the insertion section 24. The endoscopic image is transmitted via the cover glass 64 and the optical system 74 to the solid-state imaging element 78. The solid-state imaging element 78 performs photoelectric conversion on the endoscopic image, generating an electric signal. The electric signal is supplied to the video processor 18 via the hermetic connector 66, camera table 34, plug 34a (shown in FIG. 1) and receptacle 18a (shown in FIG. 1). The video processor 18 converts the electric signal to a video signal, which is supplied to the monitor 20. The monitor 20 display the endoscopic image represented by the video signal.

If the endoscopic image displayed on the monitor 20 is out of focus, the operator may rotate the focus ring 102 (shown in FIG. 2) to adjust the position of the focus. As the focus ring 102 is rotated, the cam mechanism (not shown) moves the movable part 104 moves back or forth along the optical axis. As the movable part 104 so moves, the lens frame 72 moves back or forth along the optical axis by virtue of the magnetic coupling force acting between the focusing magnets 90b and the lens-frame magnets 90a. The focus adjustment is thereby accomplished in the endoscope.

How the switch device 52 operates to make the endoscopic image brighter on the monitor 20.

Until the operation unit 112 is pushed with respect to the outer shell 44, as long as the unit 112 remains in the condition (at the first position) as shown in FIG. 3A, the first magnet 114 and the second magnet 128 opposed to each other are spaced apart by a long distance. The repulsive force generated between first magnet 114 and the second magnet 128 is very small. In this case, the leaf spring (not shown) provided in the tactile switch 122 receives only the weight of the second magnet 128 and the very small repulsive force. Thus, the tactile switch 122 is set in off state, due to the bias of the leaf spring.

Thereafter, the operator may push the operation unit 112 with respect to the outer shell 44 as is illustrated in FIG. 3B. The operation unit 112 is deformed, and the first magnet 114 approaches the second magnet 128. The more the first magnet 114 approaches the second magnet 128, the greater the repulsive force between the first magnet 114 and the second magnet 128 increases. As the repulsive force increases, the second magnet 128 applies not only its weight, but also the repulsive force, to the leaf spring provide in the tactile switch 122. The tactile switch 122 is therefore pushed and turned on, notwithstanding the bias of the leaf spring provided in it.

The signal generated when the tactile switch 122 is turned on is supplied to the video processor 18 via the rigid board 124, second connector 92b, first connector 92a, signal-transmitting circuit 80, hermetic connector 66, camera cable 34, plug 34a and receptacle 18a. The video processor 18 processes the signal. The signal processed is supplied via the cable 36 to the monitor 20. As a result, the endoscopic image displayed on the monitor 20 becomes brighter.

When the operator stops pushing the operation unit 112 with a finger, the operation unit 112 restores its initial state (first position) by virtue of the elasticity it has. The operation unit 112 quickly assumes its initial state, thanks to the repulsive force acting between the first magnet 114 and the second magnet 128. Then, the repulsive force between the first magnet 114 and the second magnet 128 gradually decreases. The tactile switch 122 is therefore turned off because of the bias of the leaf spring provided in it. At this point, the brightness of the endoscopic image is maintained. When the operation unit 112 is pushed for another time, the tactile switch 122 is turned on, whereby the endoscopic image is further increased by the prescribed value.

This sequence of operation is repeated until the endoscopic image attains the upper limit of a preset brightness range. Then, the operation unit 112 may be pushed again, whereby the tactile switch 122 is turned on. At this time, the brightness of the endoscopic image decreases to the lower limit of the present brightness range. Thereafter, the operation unit 112 may be repeatedly pushed. Every time the unit 122 is pushed, the endoscopic image becomes brighter by a prescribed value.

The operating mode of the tactile switch 122 can be set as follows. When the operation unit 112 is pushed after the endoscopic image has attained a predetermined brightness by repeatedly pushing the unit 112, the tactile switch 122 is depressed and turned on. At this time, the endoscopic image becomes darker by the prescribed value. Thereafter, as the operation unit 112 is repeatedly pushed, the endoscopic image becomes gradually darker, each time by the prescribed value. If the operation unit 112 is repeatedly pushed after the endoscopic image has become the darkest, the endoscopic image will become brighter, each time by the prescribed value.

### (Advantage of The Embodiment)

As has been described above, the following advantageous effect can be obtained by the present embodiment.

As described above, the repulsive force between the first magnet 114 and the second magnet 128 can be changed in the embodiment, by pushing the operation unit 112 with respect to the outer shell 44.
Therefore, the repulsive force that changes between the first magnet 114 and the second magnet 128 depresses the tactile switch 122, which is thereby turned on or off. As a result, the switch device 52 can be turned on and off.

The tactile switch 122 so operating is arranged in the airtight unit 46. The degradation of the tactile switch 122 can therefore be prevented even while the endoscope system 10 is undergoing a process such as autoclave sterilization. This increases the lifetime of the image-pickup device 14 for use in endoscopes. That is, the switch unit would not degrade during the autoclave sterilization, thus lengthening the lifetime of the medical apparatus, because the switch unit, i.e., an electric component, is provided in the airtight unit. In addition, the operation unit 112, which has an elastic member, can restore its initial state when released from a pressing force. Hence, the switch device 52 has good operability.

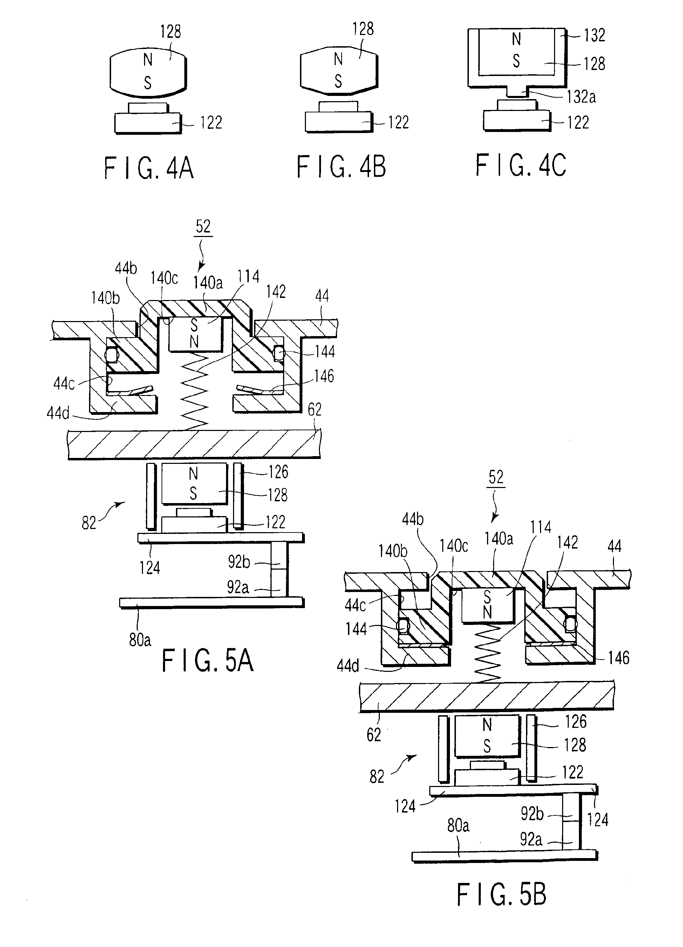

It is desired that the second magnet 128 be shaped as shown in FIGS. 4A to 4C show the positional relation the tactile switch 122 and the second magnet 128 have, not showing all other components.

The second magnet 128 shown in FIG. 4A has a curved lower surface, which may contact the tactile switch 122. Thus, this second magnet 128 can make a point contact with the tactile switch 122. The force pressing the tactile switch 122 can be concentrated. This efficiently turns on and off the tactile switch 122 on.

The second magnet 128 shown in FIG. 4B is chamfered at the peripheral part, reducing the area at which the magnet contacts the tactile switch 122.

The second magnet 128 shown in FIG. 4C is set in a magnet case 132 having a projection 132a. The projection 132a extends downwards from the lower surface of the case 132 and can therefore serve turn on and off the tactile switch 122.

The video processor 18 shown in FIG. 1 can be remote-controlled by the output of the tactile switch 122, in order to perform various functions, such as adjusting the brightness of the endoscopic image and freezing the endoscopic image. Only one switch device 52 is provided (see FIGS. 3A and 3B). Nevertheless, two or more switch devices may be provided in the embodiment.

Further, the switch device 52 may be integrally formed with a remote-switch unit, a surgical hand-piece unit or the like, in the same way as it is integrally with the image-pickup device 14 as explained above. Moreover, the endoscope 12 and the image-pickup device 14 may be combined into a single unit. In this case, the solid-state imaging element 78 may be provided in the distal end of the insertion section 24 of the endoscope 12.

### [Second Embodiment]

The second embodiment will be described with reference to FIGS. 5A and 5B. The embodiment is a modification of the first embodiment. The components identical to those of the first embodiment are designated by the same reference numbers and will not be described in detail.

### (Configuration)

As FIGS. 5A and 5B show, the switch device 52 according to the second embodiment differs from its counterpart of the first embodiment, in the arrangement of the outer shell 44, operation unit 112 and first magnet 114. The switch device 52 has, instead of the operation unit 112, an operation unit 140 made of material that can hardly deform. In addition, the switch device 52 has an elastic member (return mechanism) 142 such as a coil spring, and a watertight sealing member 144 such as an O-ring.

The operation unit 140 includes a pushing part 140a and a flange part 140b. The pushing part 40a has a recess 140c made in the inner surface. The recess 140c opens to the outer circumferential surface of the airtight-unit body 62 of the airtight unit 46. The first magnet 114 is fitted in the recess 140c. The first magnet 114 faces the second magnet 128 across the airtight-unit body 62. The magnets 114 and 128 are so arranged that they repel each other.

The outer shell 44 has a through hole 44b. A holding part 44d is formed in the outer shell 44, holding the operation unit 140 and allowing the same to move. The holding part 44d and the through hole 44b have a common axis. Therefore, the operation unit 140 can move for some distance between the inner circumferential surface of the outer shell 44 and the holding part 44d of the outer shell 44. A leaf spring 146 is secured to the holding part 44d, to receive the operation unit 140. The leaf spring 146 is shaped like a disc and has a through hole made in the center. When the operation unit 140 is depressed, it pushes the leaf spring 146. When pushed, the leaf spring 146 deforms, giving a sense of click to the operator who is operating the operation unit 140.

The elastic member 142 is, for example, a coil spring. The elastic member 142 is shorter than its natural length as in most cases (see FIG. 5A). It biases the first magnet 114 onto the outer part of the operation unit 140 (that is, away from the axis of the outer shell 44).

The watertight sealing member 144 is, for example, an O-ring. The member 144 achieves watertight sealing between the outer circumferential surface of the flange part 140b of the operation unit 140 and the inner circumferential surface of the holding part 44d of the outer shell 44. The interior of the outer shell 44 is therefore watertight.

### (Operation)

Assume that a function of increasing the brightness of endoscopic images is assigned to the switch device 52. It will be explained, based on this assumption, how the switch device 52 operates to increase the brightness of the endoscopic image displayed on the monitor 20.

As shown in FIG. 5A, the first magnet 114 and the second magnet 128 are spaced apart until the operation unit 140 is pushed. The repulsive force generated between first magnet 114 and the second magnet 128 is smaller that the bias of the leaf spring (not shown) provided in the tactile switch 122. Therefore, the tactile switch remains off.

Thereafter, the operation unit 140 may be pushed against the restoring force of the elastic member 142 as shown in FIG. 5B. Then, the distance between the first magnet 114 and the second magnet 128 decreases. The repulsive force between the first magnet 114 and the second magnet 128 therefore increases gradually. When the repulsive force becomes greater than the bias of the leaf spring (not shown) provided in the tactile switch 122, the second magnet 128 moves along the guide 126, pushing the tactile switch 122. The tactile switch 122 is thereby turned on.

When turned on, the tactile switch 122 generates a signal. This signal is transmitted to the first connector 92a via the rigid board 124 and second connector 92b. The signal is supplied from the first connector 92a to the video processor 18 via the signal-transmitting circuit 80, hermetic connector 66, camera cable 34, plug 34a and receptacle 18a. The video processor 18 processes the signal. The signal processed is supplied via the cable 36 to the monitor 20. As a result, the endoscopic image displayed on the monitor 20 becomes brighter by the prescribed value.

When the operator stops pushing the pushing part 140a of the operation unit 140 with a finger, the operation unit 140 and the first magnet 114 return to their respective initial positions (see FIG. 5A), by virtue of the elastic member 142. At this time, the operation unit 140 and the first magnet 114 quickly return to their respective initial positions, thanks to the repulsive force between the first magnet 114 and the second magnet 128. As a result, the repulsive force between the first magnet 114 and the second magnet 128 decreases. The tactile switch 122 is therefore turned off because of the bias of the leaf spring provided in it. When the operation unit 140 is pushed for another time, the brightness of the endoscopic image is further increased by a prescribed value.

### (Advantage of The Embodiment)

In the embodiment, the tactile switch 122, which is an electric component, is arranged in the airtight unit 46. The degradation of the tactile switch 122 can therefore be prevented even while the endoscope system 10 is undergoing a process such as autoclave sterilization. This increases the lifetime of the image-pickup device 14 for use in endoscopes. In addition, the operation unit 140 can automatically return to the initial position when the operator stops pushing it, because it is pressed by the elastic member 142 via the first magnet 114. The tactile switch 122 is therefore readily turned off. Hence, the switch device 52 has good operability.

### [Third Embodiment]

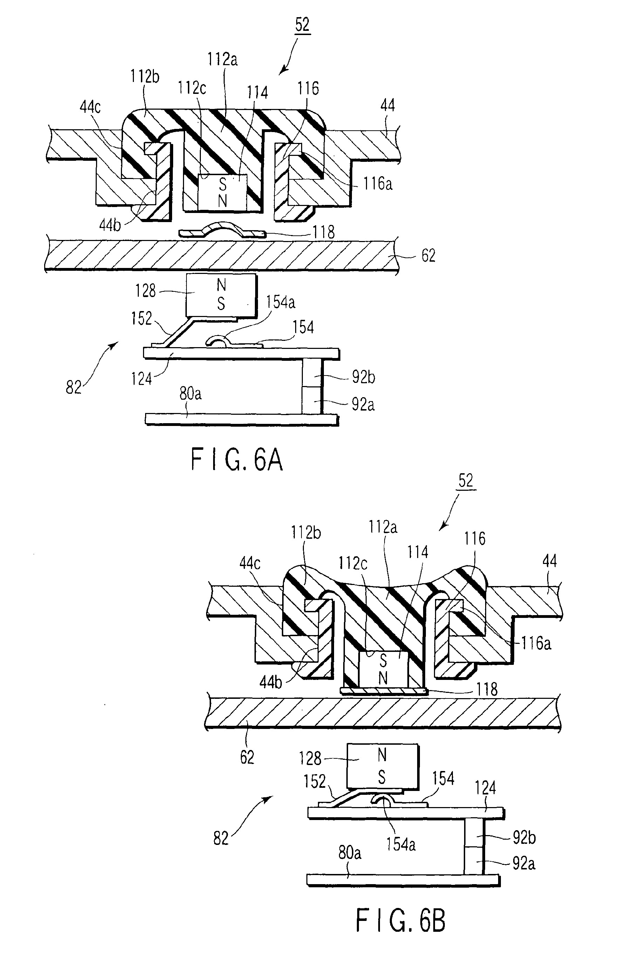

The third embodiment will be described with reference to FIGS. 6A and 6B. The embodiment is a modification of the first embodiment. The components identical to those of the first embodiment are designated by the same reference numbers and will not be described in detail.

### (Configuration)

As FIGS. 6A and 6B show, the third embodiment differs from the first embodiment in the components of the switch unit 82.

The switch unit 82 has a first contact (switch part) 152 and a second contact (switch part) 154 on one side o the rigid board 124, instead of the tactile switch 122. The first contact 152 and second contact 154 constitute a switch unit that has electric contacts.

The first contact 152 is shaped like a leaf spring. The first contact 152 has elasticity and is secured to the second magnet 128 with adhesive or the like (not shown), forming an integral unit together with the second magnet 128.

At least one of the contacts 152 and 154 (the second contact 154 in the embodiment) has a projection 154a so that the contacts 152 and 154 may be electrically connected with high reliability.

### (Operation)

Assume that a function of increasing the brightness of endoscopic images is assigned to a remote switch device 52. It will be explained, based on this assumption, how the remote switch device 52 operates to increase the brightness of the endoscopic image displayed on the monitor 20.

As shown in FIG. 6A, the first magnet 114 and the second magnet 128 are spaced apart until the operation unit 112 is pushed. The repulsive force generated between the two magnets 114 and 128 is smaller that the bias of the first contact 152. Therefore, the first contact 152 and the second contact 154 remain not electrically connected to each other (they remain off).

Thereafter, as shown in FIG. 6B, the operator may push the operation unit 112 with a finger, the operation unit 112 is deformed, whereby the distance between the first magnet 114 and the second magnet 128 decreases. The repulsive force between the first magnet 114 and the second magnet 128 therefore increases gradually. When the repulsive force becomes greater than the bias of the first contact 152, the first contact 152 is deformed, and the projection 154a of the second contact 154 is electrically connected to the first contact 152. The switch unit 82 is thereby turned on.

When turned on, the switch unit 82 generates a signal. The signal is supplied from the second contact 154 to the video processor 18 via the rigid board 124, second connector 92b, first connector 92a, signal-transmitting circuit 80, hermetic connector 66, camera cable 34, plug 34a and receptacle 18a. The video processor 18 processes the signal. The signal processed is supplied via the cable 36 to the monitor 20. As a result, the endoscopic image displayed on the monitor 20 becomes brighter by a prescribed value.

When the operator stops pushing the operation unit 112 with a finger, the operation unit 112 returns to its initial position (first position), by virtue of the elastic force it has. At this time, the repulsive force acting between the first magnet 114 and the second magnet 128 helps the operation unit 112 to restore its initial state. Therefore, the first contact 152 and the second magnet 128 leave the second contact 154. The switch unit 82 is therefore turned off. When the operation unit 140 is pushed for another time, the brightness of the endoscopic image is further increased by a prescribed value, in the same way as described above.

### (Advantage of The Embodiment)

As described above, in the third embodiment, the first contact 152 and the second contact 154, which are electric components, are arranged in the airtight unit 46. The degradation of the switch unit 82 can therefore be prevented even while the endoscope system 10 is undergoing a process such as autoclave sterilization. This increases the lifetime of the image-pickup device 14 for use in endoscopes.

Further, the operation unit 112 is an elastic member, and the first and second contacts 152 and 154 are elastic, too. When the operator stops pushing the operation unit 112, the unit 112 can automatically restore its initial state by virtue of the bias of the first and second contacts 152 and 154. Hence, the switch device 52 has good operability.

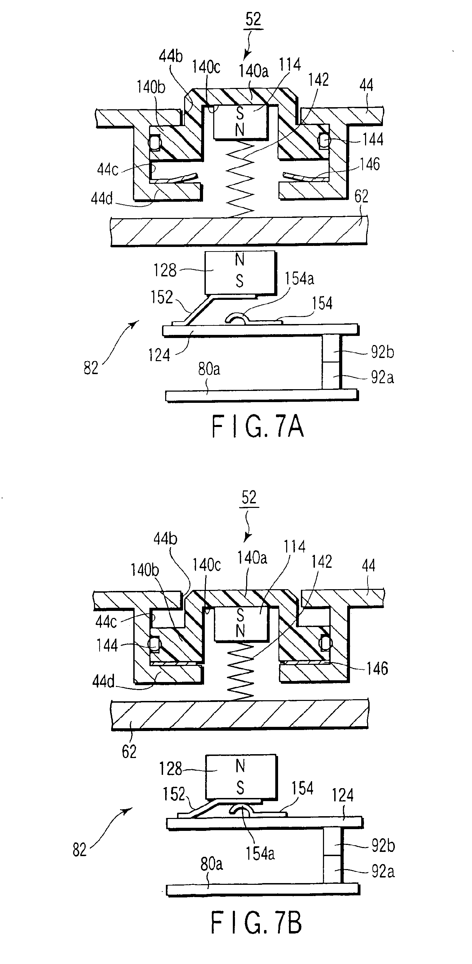

It is preferred that, as shown in FIGS. 7A and 7B, the switch unit 52 of the embodiment should be used in combination with the operation unit 140 described in conjunction with the second embodiment, though not explained here in detail.

### [Fourth Embodiment]

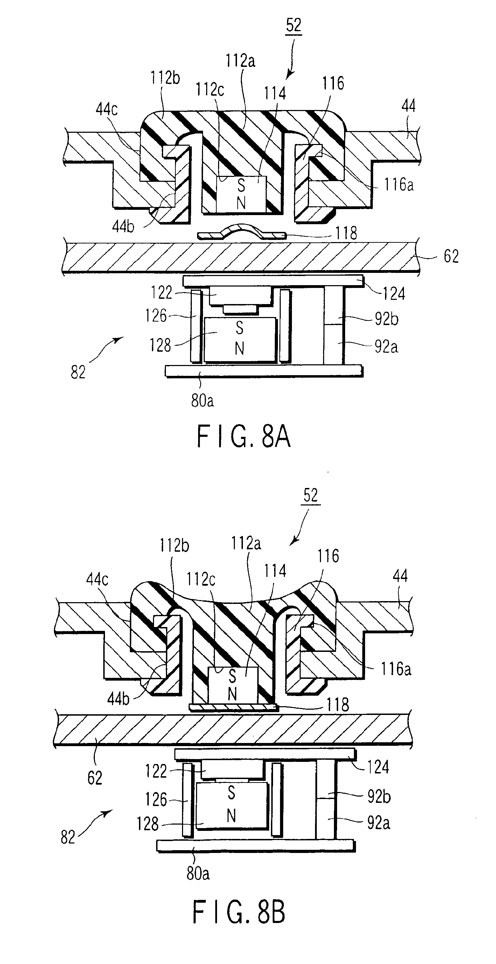

The fourth embodiment will be described with reference to FIGS. 8A and 8B. The embodiment is a modification of the first embodiment. The components identical to those of the first embodiment are designated by the same reference numbers and will not be described in detail.

### (Configuration)

As FIGS. 8A and 8B show, the fourth embodiment differs from the first embodiment in the components of the switch unit 82.

The switch unit 82 has a tactile switch 122 and a second magnet 128, which are arranged between the hard part 80a of the signal-transmitting circuit 80 and the other side of the rigid board 124. The tactile switch 122 is mounted, at the other side, on the rigid board 124. The second magnet 128 is provided on the hard part 80a of the signal-transmitting circuit 80 and held by a leaf spring (not shown), a coil spring (not shown) or the like (a leaf spring in the embodiment). The second magnet 238 can move along the along the guide 126, pushing the tactile switch 122. The first magnet 114 and the second magnet 128 face each other across the airtight-unit body 62. The magnets 114 and 128 are so arranged that they attract each other. For example, the N pole of the first magnet 114 and the S pole of the second magnet 128 are opposed to each other.

### (Operation)

Assume that a function of increasing the brightness of endoscopic images is assigned to the remote switch device 52. It will be explained, based on this assumption, how the remote switch device 52 operates to increase the brightness of the endoscopic image displayed on the monitor 20.

As shown in FIG. 8A, the first magnet 114 and the second magnet 128 are spaced apart until the operation unit 112 is pushed. The coupling force (attractive force) generated between the two magnets 114 and 128 is smaller that the bias of the leaf spring provided in the tactile switch 122. Therefore, the tactile switch 122 remains off.

Thereafter, the operator may push the operation unit 112 with a finger as shown in FIG. 8B. The operation unit 112 is therefore deformed, whereby the distance between the first magnet 114 and the second magnet 128 decreases. The coupling force between the first magnet 114 and the second magnet 128 therefore increases gradually. When the coupling force becomes greater than the bias of the leaf spring provided in the tactile switch 122, the tactile switch 122 is turned on.

When turned on, the tactile switch 122 generates a signal. The signal is supplied to the video processor 18 via the rigid board 124, second connector 92b, first connector 92a, signal-transmitting circuit 80, hermetic connector 66, camera cable 34, plug 34a and receptacle 18a. The video processor 18 processes the signal. The signal processed is supplied via the cable 36 to the monitor 20. As a result, the endoscopic image displayed on the monitor 20 becomes brighter by a prescribed value.

When the operator stops pushing the operation unit 112 with a finger, the operation unit 112 restores its initial state by virtue of the elastic force it has, against the attractive force acting between the first magnet 114 and the second magnet 128. The attractive force acting between the first magnet 114 and the second magnet 128 therefore decreases. As a result, the second magnet 128 leaves the tactile switch 122 (that is, the operation unit 112 is released from the pushed state). The tactile switch 122 is therefore turned off. When the operation unit 122 is pushed for another time, the brightness of the endoscopic image is further increased by the prescribed value, in the same way as described above.

### (Advantage of The Embodiment)

In the embodiment, the tactile switch 122, which is an electric component, is arranged in the airtight unit 46. The degradation of the tactile switch 122 can therefore be prevented even while the endoscope system 10 is undergoing a process such as autoclave sterilization. This increases the lifetime of the image-pickup device 14 for use in endoscopes. In addition, the operation unit 112 can automatically return to the initial position when the operator stops pushing it, because it is constituted by an elastic member. The tactile switch 122 is therefore readily turned off. Hence, the switch device 52 has good operability.

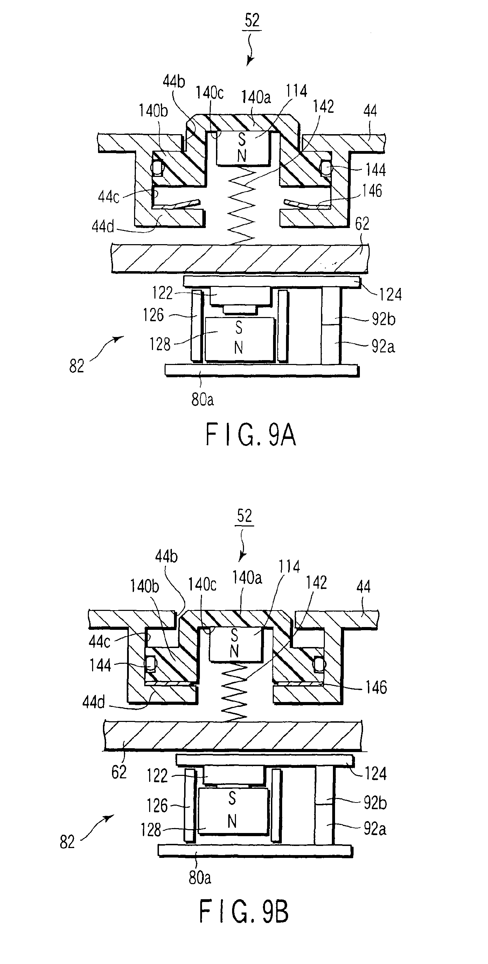

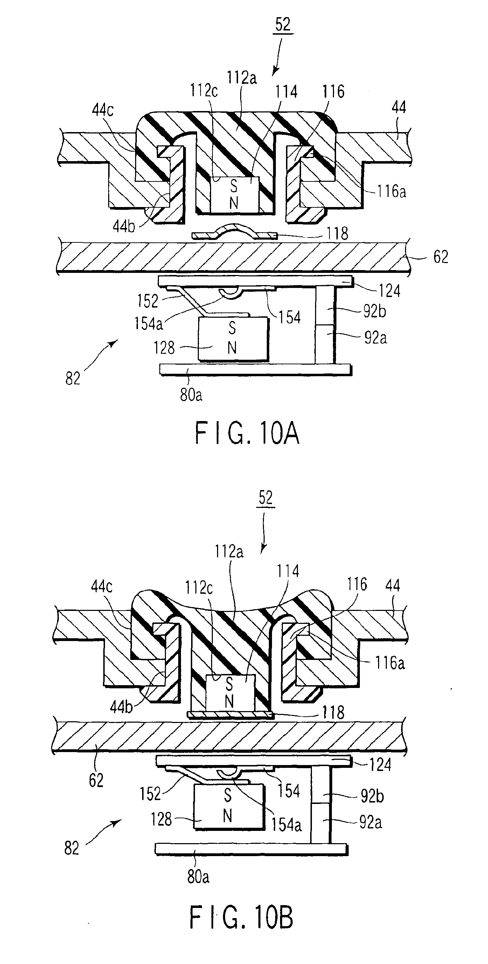

As shown in FIGS. 9A to 11B, the configurations of the second and third embodiments may be applied to the present embodiment, though not explained here in detail.

### [Fifth Embodiment]

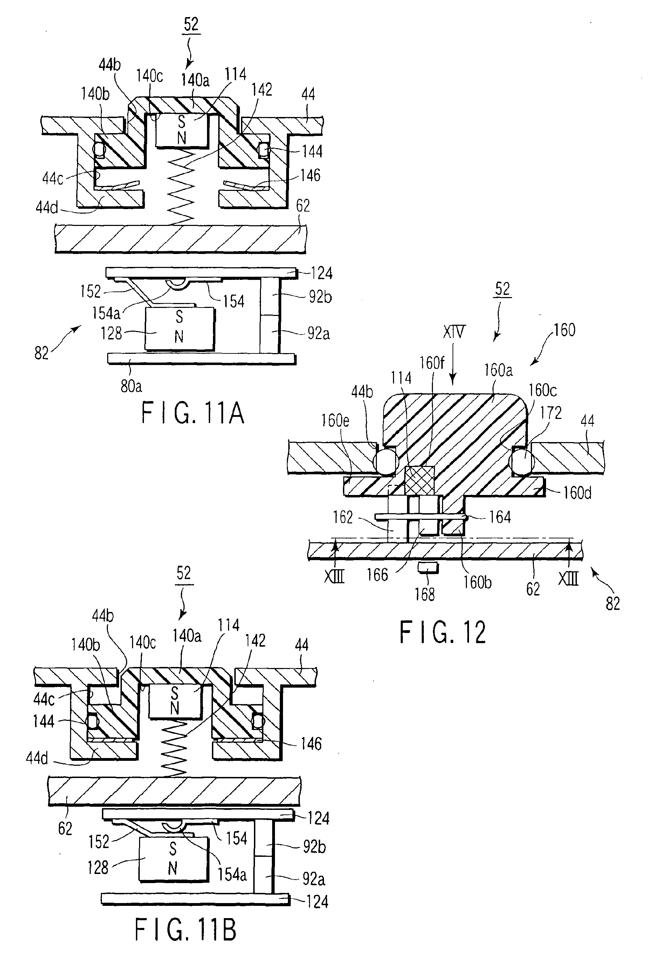

The fifth embodiment will be described with reference to FIGS. 12 to 14C. The embodiment is a modification of the first embodiment. The components which are identical to, or perform the same function as, those of the first embodiment are designated by the same reference numbers and will not be described in detail.

### (Configuration)

As FIG. 12 shows, the remote switch unit 52 includes a switch unit 82, an operation unit (magnetic-force changing mechanism) 160, a magnet 114, fastening pins (magnetic-force changing mechanism) 162, a torsion spring (magnetic-force changing mechanism) 164 and pushing parts 166. The switch unit 52 has a pair of magnetic switches 168 in place of the tactile switch 122. The magnetic switches 168 are mounted on the rigid board 124 and are provided in the airtight unit 46. They are turned on or off when a Hall IC, for example, detects a magnetic force equal to or greater than a predetermined value. The switch unit 52 has other components, which are not shown for simplicity of the drawing.

The operation unit 160 includes an operation-unit body 160a having a center shaft 160b, a recess 160c and a flange part 160d. The operation-unit body 160a can rotate around the center shaft 160b. The recess 160c is cut in the circumferential surface of the operation-unit body 160a. The flange part 160d prevents the operation-unit body 160a from slipping from the interior of the outer shell 44. The flange part 160d has a projection 160e that protrudes outwards from the flange part 160d. A recess 160f is made in the lower surface of the operation-unit body 160a, at a position deviated from the center shaft 160b. In this recess 160f, the magnet 114 is fitted and is integrally formed with the body 160a by means of adhesion or molding.

An O-ring (watertight sealing member) 172 is fitted in the recess 160c made in the circumferential surface of the operation unit 160. The O-ring 172 achieves watertight sealing between the through hole 44b of the outer shell 44 and the outer circumferential surface of the operation unit 160. Not only the interior of the outer shell 44, but also the junction between the outer shell 44 and the airtight unit 46 are watertight.

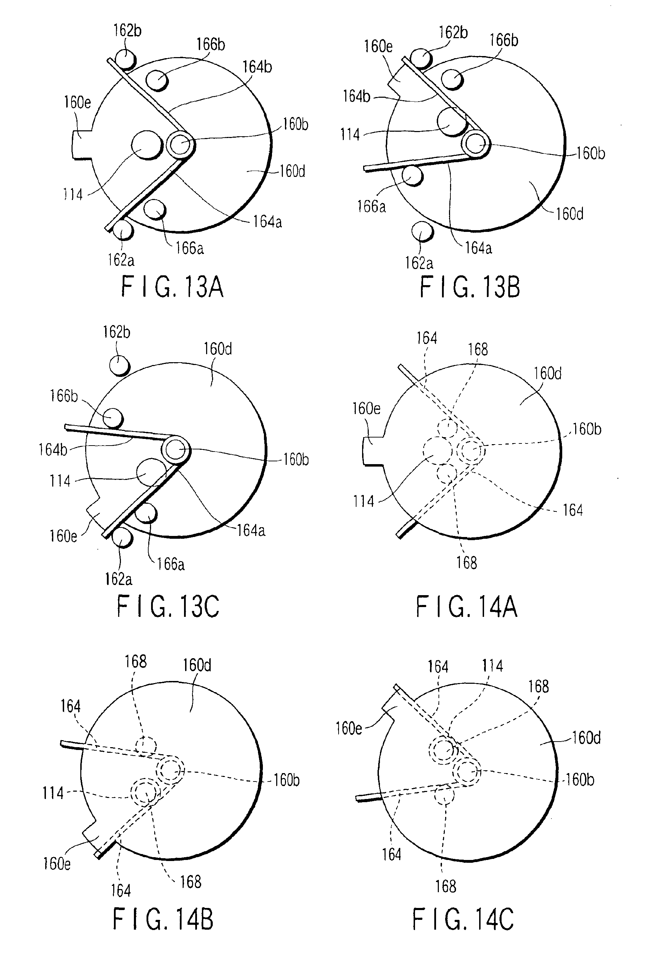

The torsion spring 164 is wound around the center shaft 160b. The torsion spring 164 are held at both ends by the fastening pins 162 that are secured to the airtight-unit body 62 by means of, for example, screw engagement. The torsion spring 164 is thus tightened a little (so that it may restore its initial shape to exert a force). The torsion spring 164 is the return means (return mechanism) for automatically rotating the operation unit 160 to the initial position thereof (see FIGS. 13A and 14A).

### (Operation)

Assume that a function of increasing the brightness of endoscopic images is assigned to the remote switch unit 52. It will be explained, based on this assumption, how the remote switch unit operates to increase the brightness of the endoscopic image displayed on the monitor 20.

In the initial state, or until the operation unit 160 is operated, the magnetic switches 168 are deviated in position from the magnet 114, as shown in FIGS. 13A and 14A. Therefore, the magnetic switches 168 cannot detect a magnetic force equal to or greater than the predetermined value. As a result, the outputs of the magnetic switches 168 remain off.

The operation unit 160 shown in FIG. 12 is rotated counterclockwise around the center shaft 160b (see FIG. 14A and FIG. 14B). Then, as shown in FIG. 13B, the first pushing part 166a pushes one end 164a of the torsion spring 164. The torsion spring 164 is therefore tightened until the projection 160e of the flange part 160d abuts on the second fastening pin 162b. When the projection 160e abuts on the pin 162b, the magnetic switches 168 face the magnet 114 as shown in FIG. 14B. The magnetic switches 168 therefore detect a magnetic force equal to or greater than the predetermined value. Hence, the magnetic switches 168 are turned on. As a result, the endoscopic image displayed on the monitor 20 becomes brighter by a prescribed value.

When the operator stops operating the operation unit 160 (that is, when he or she leaves the operation unit 160), the operation unit 160 automatically returns to its initial position by virtue of the restoring force (bias) of the torsion spring 164. At this time, the magnetic switches 168 can no longer detect the magnetic force emanating from the magnet 114, because the magnetic switches 168 are deviated in position from the magnet 114. The magnetic switches 168 are therefore turned off. The operation unit 160 may be rotated counterclockwise again. Then, the endoscopic image becomes still brighter by the prescribed value, in the same way as described above.

Similarly, the operation unit 160 shown in FIG. 12 may be rotated in the clockwise around the center shaft 160b (see FIG. 14A and FIG. 14C). Then, as shown in FIG. 13C, the second pushing part 166b pushes the other end 164b of the torsion spring 164. The torsion spring 164 is therefore tightened until the projection 160e of the flange part 160d abuts on the first fastening pin 162a. When the projection 160e abuts on the pin 162a, the magnetic switches 168 face the magnet 114 as shown in FIG. 14C. The magnetic switches 168 therefore detect a magnetic force equal to or greater than the predetermined value. Hence, the magnetic switches 168 are turned on. At this point, the video processor 18 is operated so that the endoscopic image may become brighter or less brighter by the prescribed value.

When the operator stops operating the operation unit 160 (that is, when he or she leaves the operation unit 160), the operation unit 160 automatically returns to its initial position by virtue of the restoring force (bias) of the torsion spring 164. At this time, the magnetic switches 168 can no longer detect the magnetic force emanating from the magnet 114, because the magnetic switches 168 are deviated in position from the magnet 114. The magnetic switches 168 are therefore turned off.

### (Advantage of The Embodiment)

In the present embodiment, the magnetic switches 168, which are electric components, are arranged in the airtight unit 46. The degradation of the magnetic switches 168 can therefore be prevented even while the endoscope system 10 is undergoing autoclave sterilization. When the operator stops operating the operation unit 160, the magnetic switches 168 are turned off. The operation unit 160 automatically returns to its initial position when the magnetic switches 168 are turned off. Thus, the operation unit 160 has good operability.

### [Sixth Embodiment]

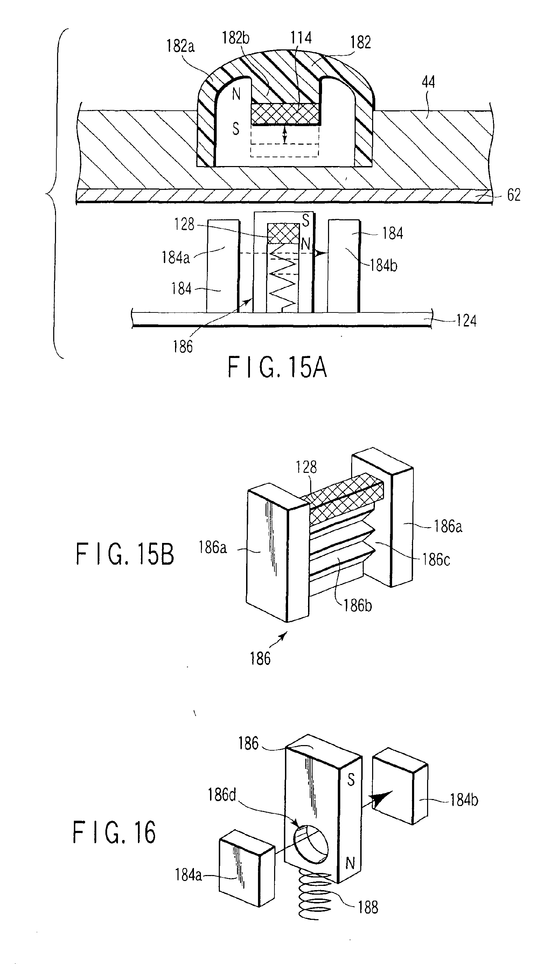

The sixth embodiment will be described with reference to FIGS. 15A and 15B. The present embodiment is a modification of the first embodiment. The components which are identical to those of the first embodiment are designated by the same reference numbers and will not be described in detail.

### (Configuration)

FIG. 15A shows a button (operation unit) 182. The button 182 includes a bending part 182a and a magnet-holding part 182b. The bending part 182a is made of elastic material such as rubber. The magnet-holding part 182b holds a first magnet 114, allowing the first magnet 114 to move up and down as the button 182 is deformed.

An optical switch 184, used in place of the tactile switch 122, is provided in the airtight package 46 and sealed by the walls of the airtight unit 46. The optical switch 184 is provided on the side of the board 124. The optical switch 184 includes a light-emitting element 184a and a light-receiving element 184b, which are spaced apart and opposed to each other. A light-cutoff member 186 is arranged between the elements 184a and 184b.

As FIG. 15B shows, the light-cutoff member 186 includes a pair of guides 186a, a curtain member 186b, and a second magnet 128. Rails 186c are provided on the guides 186a. The curtain member 186b, which is shaped like bellows and works like a spring, is interposed between the rails 186c. In other words, the curtain member 186b has its ends fitted in the rails 186a, respectively, and can expand and contract in the vertical direction. The second magnet 128 is secured to the upper edge of the curtain member 186b. The second magnet 128 is so positioned that its N and S poles facing the N and S poles of the first magnet 114, respectively.

Note that the first magnet 114 secured to the magnet-holding part 182b is samarium-cobalt magnet, neodymium magnet or the like. A rust-preventing process has been performed, as needed, on the surfaces of the first magnet 114.

### (Operation)

When the button 182 shown in FIG. 15A is pushed, the bending part 182a is deformed, whereby the magnet-holding part 182b moves down. As a result, the distance between the first magnet 114 and the second magnet 128 decreases. The magnets 114 and 128 repel each other, because of the repulsive force acting between them. The second magnet 128 moves downward along the rails 186c. As the second magnet 128 so moves, the curtain member 186b, which is secured at the upper edge to the magnet 128, moves downward, too.

At this time, the light emitted from the light-emitting element 184a reaches the light-receiving element 184b. On receiving the light, the light-receiving element 184b generates a switch-on signal.

When the operator stops pushing down the button 182, the bending part 182a of the button 182 starts restoring its initial shape by virtue of rubber elasticity. The first magnet 114 therefore moves up, decreasing the repulsive force acting between the first magnet 114 and the second magnet 128. Then, the curtain member 186b expands along the rails 186c by its elasticity, cutting off the light the light-emitting element 184a is emitting.

### (Advantage of The Embodiment)

The sensor section and electric-circuit section of the optical switch 184 are arranged in the airtight package 46 that is shielded from the steam used in autoclave sterilization. The components that are exposed to the steam are the push button 182 made of rubber and the first magnet 114 only. The embodiment can realize a switch structure that would not be degraded by autoclave sterilization.

### [Seventh Embodiment]

The seventh embodiment will be described with reference to FIG. 16. The embodiment is a modification of the sixth embodiment. The components which are identical to, or perform the same function as, those of the sixth embodiment are designated by the same reference numbers and will not be described in detail.

### (Configuration)

In the embodiment, the component that is equivalent to the light-cutoff member 186 used in the sixth embodiment is the second magnet 128. The light-cutoff member 186 (i.e., second magnet 128) has a hole 186d. The light emitted from the light-emitting element 184a can pass through the hole 186d, depending on the position the light-cutoff member 186. The magnetic poles of the light-cutoff member 186 are so positioned that the member 186 and the first magnet 114 attract each other. An elastic member (i.e., spring) 188 biases the light-cutoff member 186 upwards.

### (Operation)

When the button 182 is pushed down, the light-cutoff member 186 moves up against the bias of the spring 188, because of the magnetic force (attractive force) acting between the first magnet 114 and the second magnet 128 (i.e., light-cutoff member 186). Then, the hole 186d comes into axial alignment with the light beam coming from the light-emitting element 184a. The light-receiving element 184b therefore receives the light beam coming from the light-emitting element 184a. The switch is therefore turned on.

When the operator stops pushing the button 182 in the same way as in the sixth embodiment, the switch is turned off in the same way as in the sixth embodiment.

### (Advantage of The Embodiment)

The light-cutoff member 186 (i.e., second magnet 128) can be a comparatively large one. This can increase the magnetic force for moving the light-cutoff member 186 down as the button 182 is depressed. Thus, the magnetic force can be easily adjusted by selecting a magnet for the second magnet 128.

### [Eighth Embodiment]

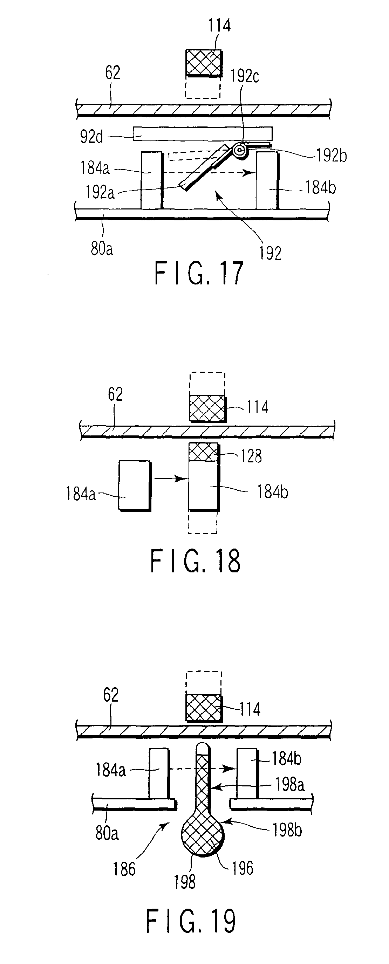

The eighth embodiment will be described with reference to FIG. 17. The embodiment is a modification of the sixth and seventh embodiments. The components which are identical to, or perform the same function as, those of the sixth and seventh embodiments are designated by the same reference numbers and will not be described in detail.

### (Configuration)

In the present embodiment, a light-cutoff member 192, which includes a magnetic plate 192a, a support shaft 192b, a torsion spring 192c and a fastening plate 192d, is provided at the part that corresponds to the light-cutoff member 186 used in the sixth embodiment. The fastening plate 92d extends parallel to the airtight-unit body 62. The support shaft 192b extends parallel to the fastening plate 192d. The torsion spring 192c is secured at one end to the fastening plate 192d. Thus, the torsion spring 192c can rotate at the other end, around the support shaft 192b.

The other end of the torsion spring 192c is biased to move away from the fastening plate 192d. The magnetic plate 192a is fixed to the other end of the torsion spring 192c. Therefore, the magnetic plate 192a inclines due to its weight if the magnetic force that the magnet 114 applies to the magnetic plate 192a is small. In this case, the magnetic plate 192a cuts off the light beam coming from the light-emitting element 184a.

### (Operation)

As the button 182 is pushed down, the magnetic force (attractive force) that the magnet 114 exerts on the magnetic plate 192a gradually increases. Therefore, as shown in FIG. 17, the magnetic plate 192a rotates around the shaft 192b against its weight and the bias of the torsion spring 192c, from the position indicated by broken line to the position indicated by solid lines. The magnetic plate 192a therefore moves and ceases to cut off the light beam coming from the light-emitting element 184a. The light beam emitted from the light-emitting element 184a is no longer cut off by the magnetic plate 192a, thus reaching the light-receiving element 184b. Hence, the switch is turned on.

When the operator stops pushing the button 182 down, the magnetic force the magnet 114 applies to the magnetic plate 192a decreases. The magnetic plate 192a inclines, due to its weight and the bias of the torsion spring 192c. The magnetic plate 192a therefore cuts off the light beam coming from the light-emitting element 184a. Thus, the switch is turned off.

### (Advantage of The Embodiment)

The present embodiment can attain the same advantage as the sixth embodiment.

### [Ninth Embodiment]

The ninth embodiment will be described with reference to FIG. 18. The embodiment is a modification of the sixth to the eighth embodiment. The components which are identical to, or perform the same function as, those of the sixth to the eighth embodiment are designated by the same reference numbers and will not be described in detail.

### (Configuration)

As FIG. 18 shows, the light-emitting element 184a is fixed in position and the light-receiving element 184b is supported to move up and down. The second magnet 128 is provided on the upper end of the light-receiving element 184b.

### (Operation)

Until the button 182 is operated, the light-receiving part of the light-receiving element 184b is located off the axis of the light beam emitted from the light-emitting element 184a (it is located at the position indicated by broken lines in FIG. 18). When the button 182 is operated (taking the position indicated by solid lines in FIG. 18), an attractive force acts between the magnets 114 and 128. Then, the light-receiving element 184b moves to a position where it can receive the light (that is, to the position indicated by solid lines in FIG. 18). As a result, the switch is turned on.

On the other hand, when the operator stops operating the button 182, the attractive force acting between the magnets 114 and 128 decreases. The light-receiving element 184b therefore moves from the position where it can receive the light (to the position indicated by broken lines in FIG. 18). The switch is therefore turned off.

### (Advantage of The Embodiment)

The present embodiment includes a few components. It can yet achieve the same advantage as the sixth to the eighth embodiment.

In the embodiment, the light-receiving element 184b can move. Nonetheless, the light-emitting element 184a may move, while the light-receiving element 184b is fixed in position.

### [Tenth Embodiment]

The tenth embodiment will be described with reference to FIG. 19. The embodiment is a modification of the sixth to the ninth embodiment. The components which are identical to, or perform the same function as, those of the sixth to the ninth embodiment, are designated by the same reference numbers and will not be described in detail.

### (Configuration)

The embodiment uses magnetic fluid 196 at a position that corresponds to the position that the curtain member 186b takes in the sixth embodiment. The magnetic fluid 196 is contained in, for example, a transparent package 198. The package 198 includes a plate-like part 198a and a magnetic-fluid reservoir 198b. The plate-like part 198a is a very thin plate and arranged between the light-emitting element 184a and the light-receiving element 184b.

The optical switch used in the embodiment, which includes elements 184a and 184b, is turned on when the light emitted from the element 184a toward the element 184b is cut off.

### (Operation)

When the button 182 is pushed down, the magnet 114 moves downwards. Then, the magnetic fluid 196 is attracted to the magnet 114, covering the plate-like part 198a. As a result, the light coming from the light-emitting element 184a is cut off, whereby the switch is turned on.

When the operator stops pushing the button 182 down, the magnet 114 moves up. The magnetic force that attracts the magnetic fluid 196 toward the magnet 114 therefore decreases. The magnetic fluid therefore flows into the magnetic-fluid reservoir 198b. Thus, the layer of magnetic fluid on the plate-like part 198a becomes thin. The light-receiving element 184b detects the light coming from the light-emitting element 184a. The switch is therefore turned off.

### (Advantage of The Embodiment)

The present embodiment includes a few components. It can yet achieve the same advantage as the sixth to the ninth embodiment.

### [Eleventh Embodiment]

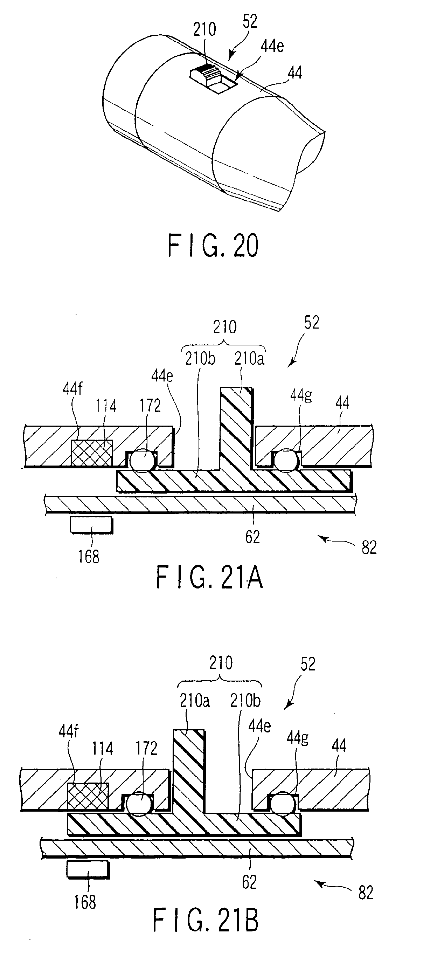

The eleventh embodiment will be described with reference to FIGS. 20 to 21B. The embodiment is a modification of the first embodiment. The components which are identical to those of the first embodiment are designated by the same reference numbers and will not be described in detail.

### (Configuration)

The eleventh embodiment differs from the first embodiment in that a slide switch (magnetic-force changing mechanism) 210 in place of the operation unit 112 of pushing type used in the first embodiment.

As FIG. 20 shows, the slide switch (operation unit) 210 is provided on the outer shell 44 of the operation section. As shown in FIGS. 21A and 21B, the slide switch 210 includes an operation knob 210a and a flange part 210b. The flange part 210b is clamped between the airtight unit casing 62 and the outer shell 44. The flange part 210b is a member than can cut off magnetic forces. The operator may slide the slide switch 210 in the lengthwise direction of an elongated hole 44e, which will be described later, in order to perform a necessary switching operation. The direction and distance in and by which the slide switch 210 can be moved is determined by the direction in which the elongated hole extends and by the length which the elongated hole has.

As FIG. 21A shows, the remote switch unit 52 includes the slide switch 210, a first magnet 114, a watertight sealing member 172, and a switch unit 82. A magnetic switch 168 is provided in the airtight package 46. The magnetic switch 168 is turned on or off when a Hall IC, for example, detects a magnetic force.

The first magnet 114 is fitted in the recess 44f made in the inner circumferential surface of the outer shell 44 and formed integral with the outer shell 44. The first magnet 144 is located so that the magnetic switch 168 may detects the magnetic force emanating from the first magnet 114.

Another recess 44g is made in the inner circumferential surface of the outer shell 44. The watertight sealing member 172 is fitted in the recess 44g, accomplishing watertight sealing between the flange part 210 of the slide switch 210 and the outer shell 44. The watertight sealing member 172 prevents the switch 210 from moving by accident.

Preferably, a plurality of magnetic switches and a plurality of magnets may be provided.

### (Operation)

When the knob 210a of the slide switch 210 is moved to the left from the position shown in FIG. 21A, the flange part 210b of the switch 210 is inserted into the gap between the magnetic switch 168 and the first magnet 114. The flange part 210b of the switch 210 therefore cuts off the magnetic force emanating from the first magnet 114 toward the magnetic switch 168. The magnetic switch 168 can no longer detects a magnetic force equal to or greater then a predetermined magnitude, applied from the first magnet 114. The output of the magnetic switch 168 therefore changes.

Preferably, the switch 210 may weaken the magnetic force applied from the first magnet 114 to the magnetic switch 168 when the switch 210 is inserted into the gap between the magnetic switch 168 and the magnet 114.

### (Advantage of The Embodiment)

The magnetic switch 168 is provided in the airtight unit 46. Hence, the degradation of the magnetic switch 168 can be prevented.

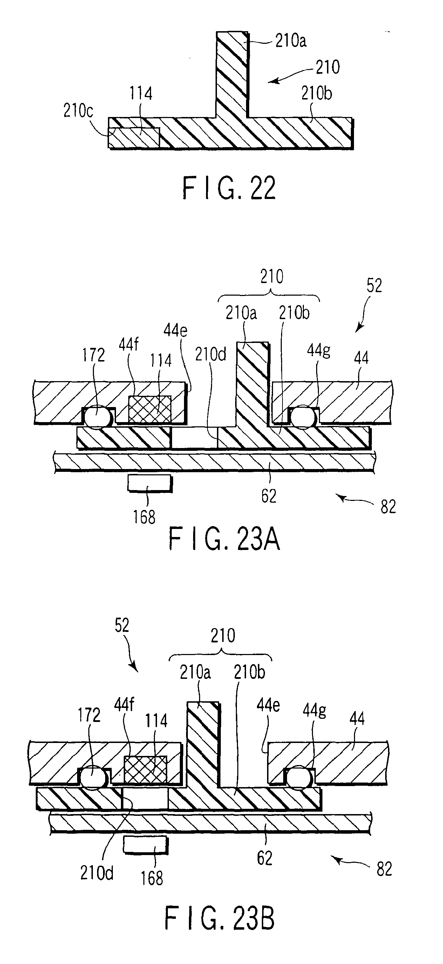

It is also desired that the switch 210 be structured as illustrated in FIG. 22. Note that the flange part 210b shown in FIG. 22 has permeability.

As FIG. 22 shows, the knob 210a of the switch 210 and the flange part 210b are made of magnetic material and can be moved along the optical axis (i.e., horizontal direction parallel to the drawing) or in the direction perpendicular to the optical axis. Alternatively, the switch 210 shown in FIG. 22 may be made of resin. The flange part 210b of the switch 210 has a recess 210c in one end. A magnetic-force cutoff plate 214 that is made of magnetic material is fitted in this recess 210c and formed integral with the flange part 210 by means of adhesion or screw fastening.

The magnetic-force cutoff plate 214 can cut off the magnetic force emanating from the magnet 114 when it enters the gap between the magnet 114 and the magnetic switch 168. The magnetic switch 168 can no longer detects the magnetic force. Hence, the switch is changed over.

The magnetic-force cutoff plate 214 can cut off the magnetic force emanating from the magnet 114 when it escapes the gap between the magnet 114 and the magnetic switch 168. The magnetic switch 168 can detect the magnetic force. Hence, the switch is changed over.

### [Twelfth Embodiment]

The twelfth embodiment will be described with reference to FIGS. 23A and 23B. The embodiment is a modification of the eleventh embodiment. The components which are identical to those of the eleventh embodiment are designated by the same reference numbers and will not be described in detail.

### (Configuration)

As FIGS. 23A and 23B show, the twelfth embodiment differs from the eleventh embodiment in the configuration of the switch 210.

The switch 210 shown in FIGS. 23A and 23B is made of magnetic material. The flange part 210b of the switch 210 has an opening 210d. The switch 210 is interposed between the magnetic switch 168 and the first magnet 114, cutting off the magnetic force emanating from the first magnet 114.

### (Operation)

When the switch 210 shown in FIG. 23A is moved to the left in the drawing, the opening 210d of the switch 210 is positioned between the magnetic switch 168 and the first magnet 114 as shown in FIG. 23B. Therefore, the magnetic force emanating from the first magnet 114 reaches the magnetic switch 168. At this time, the magnetic switch 168 detects a magnetic force equal to or greater than a predetermined magnitude. The output of the magnetic switch 168 therefore changes.

### (Advantage of The Embodiment)

The magnetic switch 168 having electric contacts is provided in the airtight unit 46. The degradation of the magnetic switch 168 can therefore be prevented.

### [Thirteenth Embodiment]

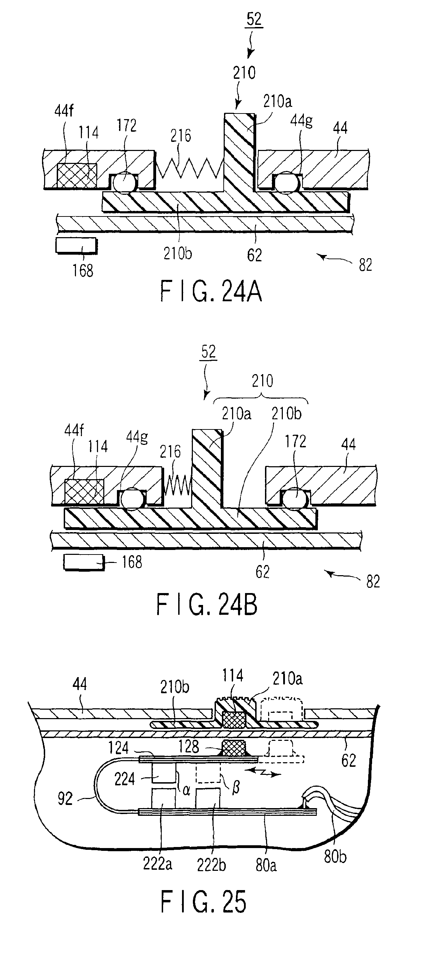

The thirteenth embodiment will be described with reference to FIGS. 24A and 24B. The embodiment is a modification of the eleventh embodiment. The components which are identical to those of the eleventh embodiment are designated by the same reference numbers and will not be described in detail.

### (Configuration)

As FIGS. 24A and 24B show, the thirteenth embodiment differs from the eleventh embodiment in that an elastic member (return mechanism) 216 is provided between the switch 210 and the elongated hole 44e. The elastic member 216 is secured at one end to the switch 210 and at the other end to the outer shell 44, with screws (not shown) or the like.

### (Operation)

The output of the magnetic switch 168 is changed over in the same way as in the eleventh embodiment, and how the switch 168 is changed over will not be explained. When the switch 210 is released, it automatically returns to its initial position, by virtue of the bias of the elastic member 216.

### (Advantage of The Embodiment)

The present embodiment can achieve the same advantage as the eleventh embodiment. In addition, it has high operability because the switch 210 automatically returns to its initial position when the operator releases the switch 210.

### [Fourteenth Embodiment]

The fourteenth embodiment will be described with reference to FIG. 25. The present embodiment is a modification of the eleventh embodiment. The components which are identical to those of the eleventh embodiment are designated by the same reference numbers and will not be described in detail.

### (Configuration)

As FIG. 25 shows, the first magnet 114 is secured near the back of the operation knob 210a of the slide switch 210 (thus, the magnet 114 faces the airtight unit casing 62). The first magnet 114 therefore moves as the slide switch 210 is operated.

An electric circuit board 80a is provided in the airtight unit casing 62. The electric circuit board 80a is a flex-rigid board. The board 80a is electrically connected to an extension board 124 by an FPC unit 92. The extension board 124 can move in the same direction the slide switch 210, as long as allowed by the length of the FPC unit 92, while remaining spaced by a prescribed displace from the inner surface of the casing 62.

Two light-receiving elements, i.e., a first element 222a and a second element 222b, are mounted on the electric circuit board 80a. A light-emitting element 224 is mounted on the extension board 124. The second magnet 128 is mounted on one side of the extension board 124. The second magnet 128 has its magnetic poles arranged so that the first magnet 114 and the second magnet 128 attract each other.

### (Operation)

When the operator operates the operation knob 210a of the slide switch 210, the first magnet 114 arranged outside the airtight unit casing 62 is moved. At this point, an attractive force acts between the first magnet 114 and the second magnet 128. The second magnet 128 provided in the airtight unit casing 62 moves, too. The extension board 124 and the light-emitting element 224 move, too, because the second magnet 128 is integrally formed with the extension board 124 and the light-emitting element 224. As a result, as the slide switch 210 is moved, the light-emitting element 224 moves between the position α indicated by solid lines and the position P indicated by broken lines, both position shown in FIG. 25.

A power-supply voltage is applied to the electric circuit board 80a (via FPC unit 92) and the extension board 124 through an electric-signal lines 80b. The light-emitting element 224 and the first and second light-receiving elements 222a and 222b are thereby driven. While the light-emitting element 224 stays at the position α, the first light-receiving element 222a receives the light emitted from the light-emitting element 224.

While the light-emitting element 224 stays at the position β, the second light-receiving element 222b receives the light emitted from the light-emitting element 224. Thus, the first light-receiving element 222a and the second light-receiving element 222b generate electric signals representing their respective light-receiving states in real time. These signals are transmitted to an apparatus (not shown) that is provided outside the airtight unit 46.

### (Advantage of The Embodiment)

The operator can change the respective light-receiving states of the first and second light-receiving elements 222a and 222b, by sliding the operation knob 210a that is provided outside the airtight unit 46. Upon receiving the electric signals through the electric-signal lines 80b, the apparatus provided outside the airtight unit 46 can perform various operations in accordance with the operation of the slide switch 210. In this switch structure, all elements not so resistant to moisture, such as the light-receiving elements 222a and 222b, are provided in the airtight unit 46. The switch structure is therefore simple and can yet greatly resistant to the steam used in autoclave sterilization.

### [Fifteenth Embodiment]

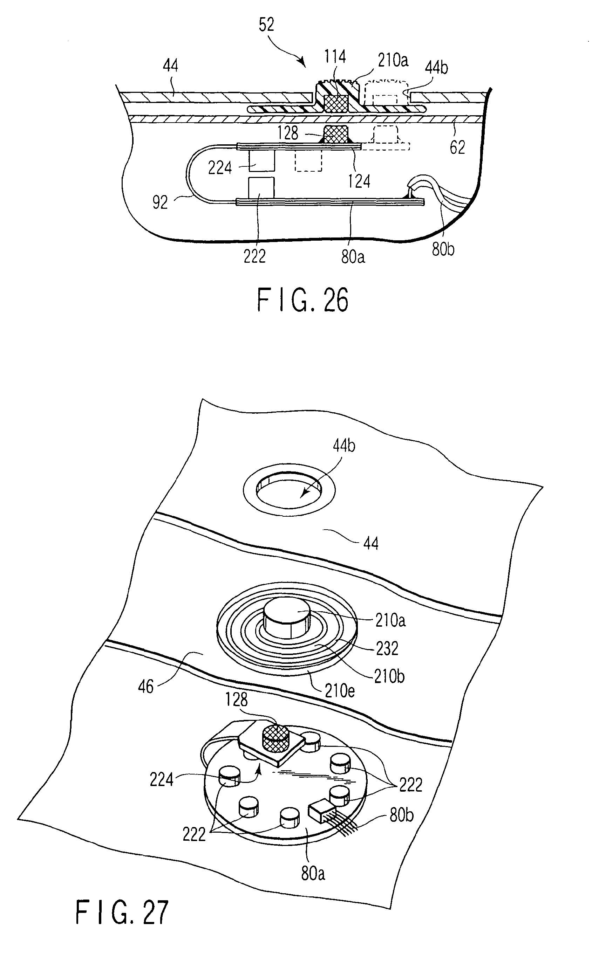

The fifteenth embodiment will be described with reference to FIG. 26. The present embodiment is a modification of the fourteenth embodiment. The components which are identical to those of the fourteenth embodiment are designated by the same reference numbers and will not be described in detail.

### (Configuration)

As FIG. 26 shows, a light-emitting element 224 is arranged on an electric circuit board 80a of the same type as used in the fourteenth embodiment. One light-receiving element 222 is arranged on the extension board 124. In any other respect, the embodiment is identical to the fourteenth embodiment.

### (Operation)

As the operator slides the operation knob 210a, the light-receiving element 222 provided on the extension board 124 moves. Hence, the light-receiving state of the light-receiving element 222 is switched between "receiving state" and "non-receiving state."

### (Advantage of The Embodiment)

The operator can change the light-receiving state of the light-receiving element 222 provided in the airtight unit 46 as he or she operates the operation knob 210a that is provide outside the airtight unit 46. Any apparatuses outside the airtight unit 46 can receive signals from the light-receiving element 222. Therefore, the switch structure of the embodiment can be simple and yet greatly resistant to the steam used in autoclave sterilization, like the switch structure of the fourteenth embodiment.

### [Sixteenth Embodiment]

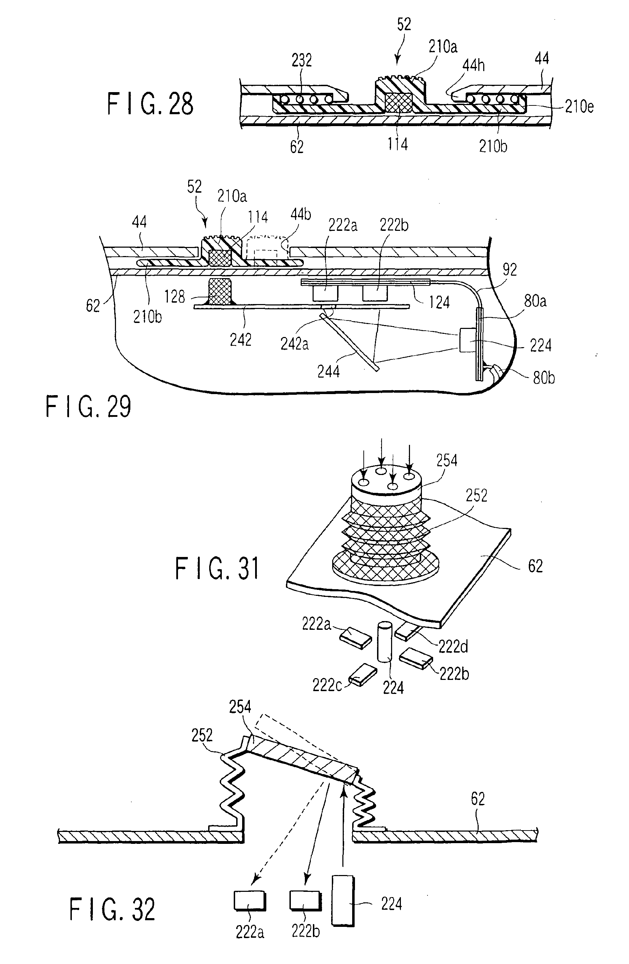

The sixteenth embodiment will be described with reference to FIG. 27 and FIG. 28. The present embodiment is a modification of the fourteenth embodiment. The components which are identical to those of the fourteenth embodiment are designated by the same reference numbers and will not be described in detail.

### (Configuration)

As FIG. 27 shows, a number of light-receiving elements 222 are arranged on the electric circuit board 80a of the same type as used in the fourteenth embodiment. On the other hand, the outer shell 44 of the airtight unit 46 has a through hole 44b that is a circular hole as in the first embodiment.

As FIG. 28 shows, a spiral spring 232 is provided between the outer shell 44 and the slide switch 210 and used as bias member. The innermost turn of the spiral spring 232 abuts on the projection 44h provided on the inner circumferential surface of the though hole (circular hole) 44b. The outermost turn of the spiral spring 232 abuts on the outer circumferential edge of the flange part 210b of the slide switch 210. In any other respect, the embodiment is identical to the fourteenth embodiment.

### (Operation)

The spiral spring 232 always biases the operation knob 210a toward the center of the through hole 44b. In this condition, the operation knob 210a assumes a neutral position. The operator may slide the operation knob 210a to any desired position in the through hole 44b. As the knob 210a is so moved, the light-emitting element 224 moves in the airtight unit 46 in the same manner as in the fourteenth embodiment. The light-receiving state of any light-receiving element 222 located near the light-emitting element 224 changes. The switch is changed over.

When released, the operation knob 210a returns, moving toward the center of the through hole 33b, by virtue of the bias of the spiral spring 232. At this point, the light-receiving state changes. The switch is therefore changed over.

### (Advantage of The Embodiment)

The operator can change the light-receiving state of the light-receiving element 222 provided in the airtight unit 46 as he or she operates the operation knob 210a that is provide outside the airtight unit 46. Since many light-receiving elements 222 are provided, the slide switch 210 can initiate various functions as it is moved. Hence, the switch can operate as a multifunction switch or a joystick. Any apparatuses outside the airtight unit 46 can receive signals from the light-receiving element 222. Therefore, the switch structure of the embodiment can be simple and yet greatly resistant to the steam used in autoclave sterilization, like the switch structure of the fourteenth embodiment.

### [Seventeenth Embodiment]

The seventeenth embodiment will be described with reference to FIG. 29. The embodiment is a modification of the fourteenth embodiment. The components which are identical to those of the fourteenth embodiment are designated by the same reference numbers and will not be described in detail.

### (Configuration)

As FIG. 29 shows, the second magnet 128 of the same type as used in the fourteenth embodiment is provided on a slit plate 242, not on the extension board 124. The slit plate 242 has a slit 242a (having any desired shape). The slit plate 242 can be moved in the same way as the extension board 124 moves in the fourteenth embodiment.

First and second light-receiving elements 222a and 222b are mounted on the extension board 124. The extension board 24 is fixed in the airtight unit casing 62. The slit plate 242 extends parallel to the extension board 124. A mirror 244 is opposed to the extension board 124 across the slit plate 242.

A light-emitting element 224 is mounted on the electric circuit board 80a. The light-emitting element 224 faces the mirror 244. The electric circuit board 80a is fixed in the airtight unit casing 62.

### (Operation)

The operator may slide the operation knob 210a. Then, the second magnet 128 and the slit plate 242 move in the same way as in the fourteenth embodiment.

When driven, the light-emitting element 224 emits light. The mirror 244 reflects the light, guiding the light toward the extension board 124. A greater part of the light is cut off by the slit plate 242. The remaining part of the light passes through the slit 242a cut in the slit plate 242. Therefore, the light reaches either the first light-receiving element 222a or the second light-receiving element 222b, which is aligned with the slit 242a.

As the slit plate 242 moves as described above, the slit 242a moves, too. As a result, the light-receiving states of the light-receiving elements 222a and 222b change in the same way as in the fourteenth embodiment. Hence, the switch is changed over.

The light emitted from the light-emitting element 224 can of course be applied directly to the first light-receiving elements 222a or the second light-receiving elements 222b, not reflected and guided by the mirror 244. That is, the mirror 244 need not be used if the electric circuit board 80a is arranged at an appropriate position.

### (Advantage of The Embodiment)

The operator may operate the operation knob 210a to change the light-receiving states of the first and second light-receiving elements 222a and 222b, in the same way as in the fourteenth embodiment. Since no component that should be moved as an electric circuit board, like the extension board 124 used in the fourteenth embodiment, no load is repeatedly exerted on, for example, the FPC unit 92. This helps to provide a simple switch structure that is greatly reliable and resistant to the steam used in autoclave sterilization.

In the embodiments described thus far, the electric circuit board 80a and the extension board 124 are electrically connected by the FPC unit 82 provided on the flex-rigid board. Nevertheless, the electric circuit board 80a and the extension board 124 need not be mounted on a flex-rigid board. Rather, they may be connected by an FPC cable, electric-signal harness, or the like. Alternatively, the FPC cable or the harness may be directly secured to separate boards by means of, for example, soldering. Still alternatively, the circuit board 80a, extension board 124 and FPC unit 92 may be combined, forming a single FPC unit.

Neither fastening means for various boards provided in the airtight unit 46 nor means for restricting the direction in which the boards should move have been described, particularly in conjunction with the present embodiment. The boards may be held by various known means and guided in specific directions by various known means. For example, they may be fastened with screws to holding frames of general type or may be loosely fitted in grooves and moved along the grooves.

### [Eighteenth Embodiment]

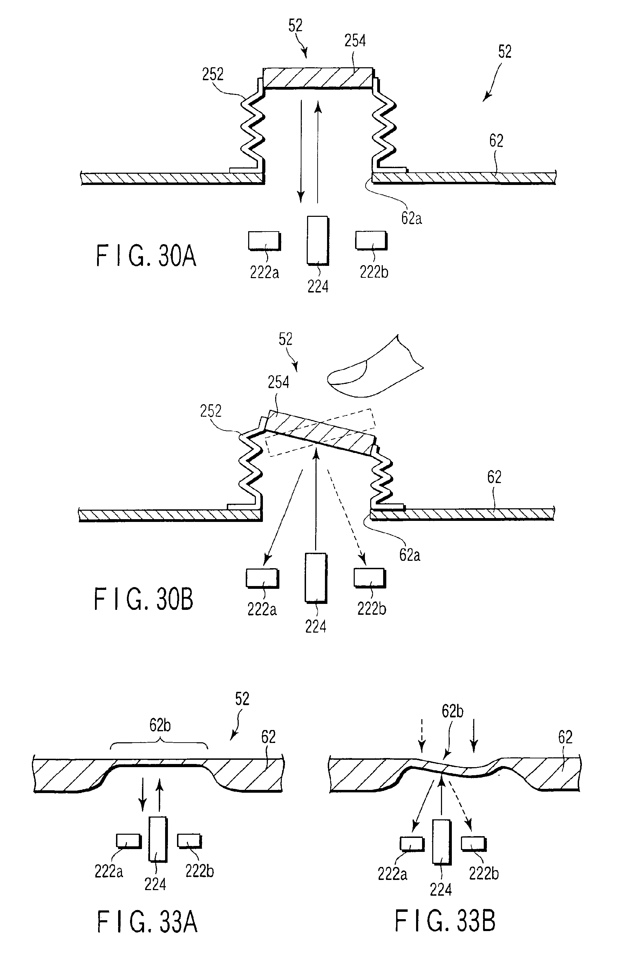

The eighteenth embodiment will be described with reference to FIGS. 30A and 30B. The embodiment is a modification of the first embodiment. The components which are identical to those of the first embodiment are designated by the same reference numbers and will not be described in detail.

### (Configuration)

As FIGS. 30A and 30B show, the switch device 52 for use in endoscopes, according to the present embodiment, includes an airtight unit casing 62 having a hole 62a, a metal bellows 252, a reflection-direction changing plate 254, a first optical sensor (first optical detecting means) 222a, a second optical sensor (second optical detecting means) 222b, and a light-emitting element (light-emitting means) 224. The metal bellows 252 can bend and deform.

The metal bellows 252 is coupled, at the proximal end, to the airtight unit casing 62 and surrounds the hole 62a. The metal bellows 252 is coupled, at the distal end, to the reflection-direction changing plate 254 in airtight fashion. Since the metal bellows 252 can deform, the reflection-direction changing plate 254 can be inclined to the airtight unit casing 62.

The first and second optical sensors 222a and 222b and the light-emitting element 224 are arranged in the airtight unit casing 62. The light-emitting element 224 is located halfway between the first and second optical sensors 222a and 222b.

### (Operation)

How the present embodiment operates will be explained.

FIG. 30A shows the switch device 52 that remains off. The light emitted from the light-emitting element 224 is reflected downwards by the reflection-direction changing plate 254. Therefore, neither the first optical sensor 222a nor the second optical sensor 222b can detect the light coming from the light-emitting element 224. Hence, the first and second optical sensors 222a and 222b remain off.

FIG. 30B shows the switch device 52 that remains on. The operator has inclined the reflection-direction changing plate 254 shown in FIG. 30A, with a finger. As shown in FIG. 30B, the reflection-direction changing plate 254 is inclined.

If the reflection-direction changing plate 254 is inclined as indicated by solid lines in FIG. 30B, the light emitted from the light-emitting element 224 is reflected by the reflection-direction changing plate 254 and applied to the first optical sensor 222a. The first optical sensor 222a is therefore turned on.

If the reflection-direction changing plate 254 is inclined as indicated by broken lines in FIG. 30B, the light emitted from the light-emitting element 224 is reflected by the reflection-direction changing plate 254 and applied to the second optical sensor 222b. In this case, the second optical sensor 222b is turned on.

### (Advantage of The Embodiment)

The number of light-emitting elements 224 can be reduced with respect to that of first and second optical sensors 222a and 222b. That is, the number of components provided in the airtight unit 46 can be decreased. This helps to make the airtight unit 46 smaller.

Since the number of light-emitting elements 224 can be reduced with respect to that of first and second optical sensors 222a and 222b, the power consumption and the manufacturing cost can be reduced.

In the present embodiment, the metal bellows 252 and the reflection-direction changing plate 254 can be inclined in two directions so that the first and second optical sensors 222a and 222b may receive light. Instead, the metal bellows 252 and the reflection-direction changing plate 254 may be inclined in four directions as shown in FIG. 31. In this case, two other optical sensors 222c and 222d are arranged around the light-emitting element 224. Further, the metal bellows 252 and the reflection-direction changing plate 254 can be inclined in more than four directions.

As described above, the light-emitting element 224 is located between the first and second optical sensors 222a and 222b in the present invention. Instead, the second optical sensor 222b may be located between the first optical sensor 222a and the light-emitting element 224, as is illustrated in FIG. 32. If this is the case, either the first optical sensor 222a or the second optical sensor 222b is activated in accordance with the inclination angle (the magnitude of input) of the reflection-direction changing plate 254. In this configuration, the plate 254 may be depressed in the same way as were the release button of the camera, performing a sequence of operations. For example, the plate 254 performs focus adjustment when half depressed and then shutter-releasing when further depressed.

### [Nineteenth Embodiment]

The nineteenth embodiment will be described with reference to FIGS. 33A and 33B. The embodiment is a modification of the eighteenth embodiment. The components which are identical to those of the eighteenth embodiment are designated by the same reference numbers and will not be described in detail.

As FIG. 33A shows, the switch device 52 for use in endoscopes, according to the present embodiment, differs from the switch according to the eighteenth embodiment in configuration of the input unit (i.e., airtight unit casing 62, metal bellows 252 and reflection-direction changing plate 254). In the embodiment, the airtight unit casing 62 is not opened and has a thin-wall part (operation part) 62b. The first and second optical sensors 222a and 222b are arranged near the thin-wall part 62b, as in the switch device 52 according to the eighteenth embodiment. That side of the thin-wall part 62b, which is close to the first and second optical sensors 222a and 222b and the light-emitting element 224, is a mirror surface.

### (Operation)

How the present embodiment operates will be explained.

The thin-wall part 62b shown in FIG. 33A may be depressed from outside the airtight casing unit 62. Then, the thin-wall part 62b is deformed and reflects the light emitted from the light-emitting element 224, guiding the light in a desired direction. Thus, the switch can perform the same function as the switch device 52 does in the eighteenth embodiment.

### (Advantage of The Embodiment)

The metal bellows 252 used in the eighteenth embodiment is unnecessary. No airtight junctions are required at the ends of the bellows 252, and the airtight casing unit 62 need not have a hole 62a. Hence, the embodiment can be simpler and made at a lower cost than the eighteenth embodiment.

### [Twentieth Embodiment]

Endoscopes are used to obtained endoscopic images. To obtain images, the insertion section of the endoscope, which is an elongate section, is inserted into a body cavity and removably attached to the eyepiece section of the endoscope. Generally, the endoscope system having the endoscope has a remote switch that performs various functions, such as adjusting the brightness of endoscopic images, freezing the endoscopic images, and adjusting the focal distance.

In recent years, autoclave sterilization that uses high-temperature, high-pressure steam has been widely used as a method of sterilizing image-pickup devices.

An image-pickup device for use in endoscopes, which can be subjected to the autoclave sterilization, is disclosed in, for example, Japanese Patent No. 3300135. This image-pickup device for use in endoscopes has a magnetic switch used as remote switch. A magnet (magnetic-force generating means) is moved toward and away from the magnetic switch, thereby turning on and off the magnetic switch.

In view of this, the present invention provides a switch device that is inexpensive and easy to operate.

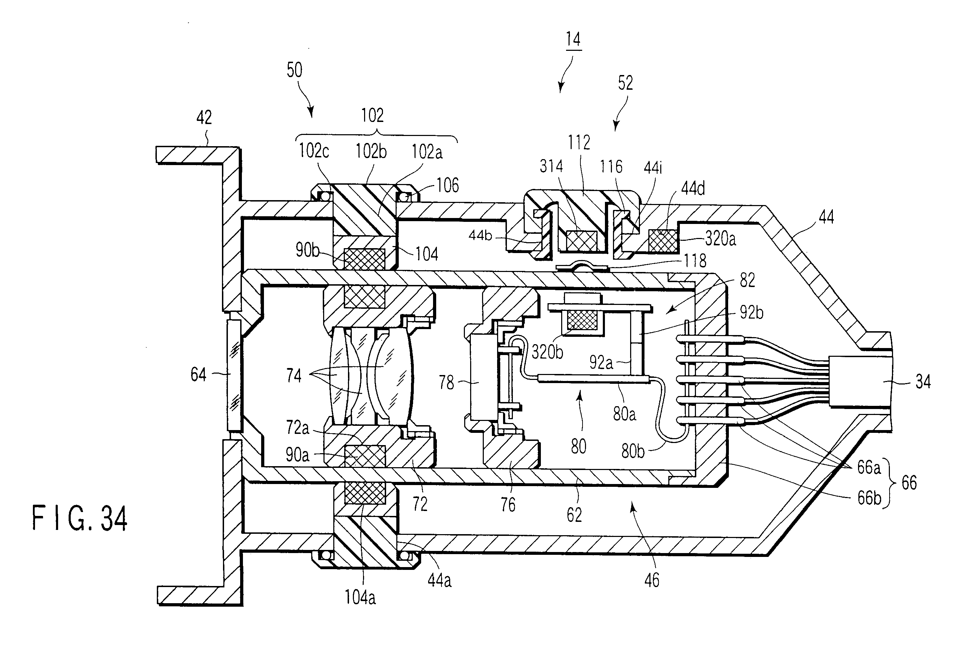

The twentieth embodiment will be described, with reference to FIGS. 34 to 35B. The embodiment is a modification of the first embodiment. The components which are identical to those of the first embodiment are designated by the same reference numbers and will not be described in detail.

### (Configuration)

An image-pickup device 14 for use in endoscopes (medical apparatuses) will be described in detail.

The image-pickup device 14 for use in endoscopes, which is shown in FIG. 34, differs from the image-pickup device 14 (see FIG. 2) in the configuration of the airtight unit 46 and switch device 52. In view of this, the other components of this image-pickup device 14 will not be described.

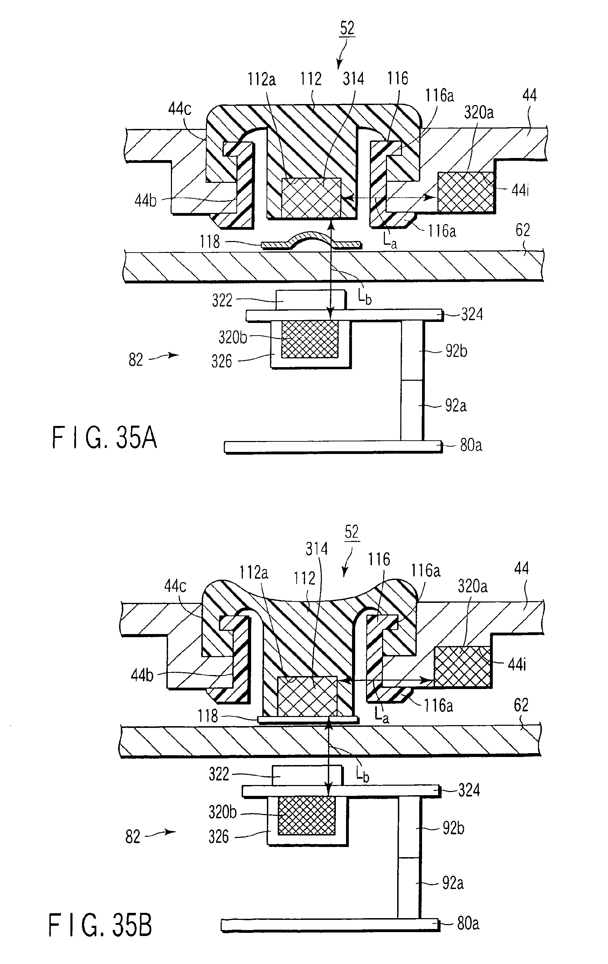

As shown in FIGS. 35A and 35B, the switch device 52 includes a switch unit 82, an input unit (operation unit) 112, a magnet (magnetic-force generating portion) 314, a fastening member 116, a leaf spring 118, and a first magnetic member (magnetic-force changing mechanism) 320a. The switch unit 82 is of the type described above.

The outer shell 44 has a through hole 44b that extends toward its axis. A recessed part 44c is provided around the through hole 44b and opens toward the axis of the outer shell 44. The input unit 112 is fitted in the recessed part 44c and passing through the through hole 44b. The recessed part 44c and the operation unit 112 are held by the fastening member 116 that is shaped like a ring and has a flange part 116a. That is, the fastening member 116 secures the operation unit 112 to the outer shell 44, preventing the operation unit 112 from slipping from the outer shell 44.

The input unit 112 is made of elastic material such as silicone rubber and is shaped like a button having a cross section that is shaped like letter T. Therefore, the input unit 112 is deformed when the operator pushes it with a finger, but restores its initial shape when the operator stops pushing it. Thus, the input unit 112 changes in position and shape, assuming the first position (see FIG. 35A) until it is pushed, and the second position (see FIG. 35B) when it is pushed. The input unit 112 has a recess 112a. In the recess 112a, the magnet 314 is fitted and integrally formed with the unit 112 by means of adhesion or molding.

The leaf spring 118 is provided on the outer circumferential surface of the airtight-unit body 62. The leaf spring 118 is deformed as the magnet 314 pushes it when the input unit 112 is operated. As it is so deformed, the leaf spring 118 gives a sense of click to the operator who is operating the input unit 112.

A recess 44i is made in the inner surface of the outer shell 44, in the vicinity of the through hole 44b and the recessed part 44c. The first magnetic member 320a is fitted in this recess 44i. The first magnetic member 320a is formed integral with the outer shell 44 by means of adhesion or the like.

As FIG. 35A and 35B show, the switch unit 82 includes a magnetic sensor (magnetic-force sensor) 322, a rigid board 324, a magnetic frame 326, and a second magnetic member (magnetic-force changing mechanism) 320b. The magnetic sensor 322 is a Hall IC or the like.

The magnetic sensor 322 is mounted on one side of the rigid board 324. On the other side of the rigid board 324, the magnetic frame 326 is secured by means of soldering or the like (solder is not shown). The second magnetic member 320b is fixed to the magnetic frame 326. The magnetic sensor 322 and the second magnetic member 320b are arranged, facing each other. The magnetic sensor 322 is provided between the magnet 314 and the second magnetic member 320b.

On the other side of the rigid board 324, a second connector 92b is provided. The second connector 92b is electrically connected to the first connector 92a. Hence, the electric signal generated when the magnetic sensor 322 is turned on or off is transmitted to the first connector 92a via the rigid board 324, second connector 92b and first connector 92a.

The magnet 314 and the first magnetic member 320a are spaced apart by a distance Lₐ. The magnet 314 and the second magnetic member 320b are spaced apart by a distance L_{b}. The distance L_{b} is longer than the distance Lₐ while the input unit 112 stays at the first position (see FIG. 35A). The distance L_{b} is shorter than the distance Lₐ while the input unit 112 stays at the second position (see FIG. 35B).

While the input unit 112 stays at the first position, magnetic fluxes converge, oriented from the magnet 314 toward the first magnetic member 320a (in the first direction) because the distance L_{b} is longer than the distance Lₐ, On the other hand, while the input unit 112 stays at the second position, the magnetic fluxes converge, oriented from the magnet 314 toward the second magnetic member 320b (in the second direction) because the distance L_{b} is shorter than the distance Lₐ. Thus, when the input unit 112 is operated, moving the magnet 314, the first magnetic member 320a and the second magnetic member 320b relatively moves toward and away from the magnet 314, respectively, or vice versa. As a result, the direction in which the magnetic fluxes emanating from the magnet 314 are oriented changes from the first direction to the second direction, or vice versa. That is, the first magnetic member 320a and the second magnetic member 320b are magnetic-flux changing means for changing the orientation of the magnetic fluxes emanating from the magnet 314, as the magnet 314 approaches or moves away.

### (Operation)

How the endoscope system 10 according to the embodiment operates will be explained, mainly as to how the switch device 52 operates.

Assume that the switch device 52 shown in FIG. 34 has the function of increasing the brightness of endoscopic images. Then, as shown in FIG. 1, the image-pickup device 14 is removably attached to the eyepiece section 26 that is provided at the proximal end of the insertion section 24 of the endoscope 12. The device 14 therefore serves to accomplish endoscopic examination.

Illumination light is applied from the light source device 16 to the insertion section 24 through the receptacle 16a, connector 32, light-guiding cable 30 and mouth ring 28. The light is emitted from the illumination lens (not shown) provided in the distal end of the insertion section 24. The light thus emitted illuminates the object. The image of the object illuminated, or an endoscopic image, is applied to the objective lens provided in the distal end of the insertion section 24. The endoscopic image is transmitted via the cover glass 64 and the optical system 74 (both shown in FIG. 34) to the solid-state imaging element 78. The solid-state imaging element 78 performs photoelectric conversion on the endoscopic image, generating an electric signal. The electric signal is supplied to the video processor 18 via the signal-transmitting circuit 80, hermetic connector 66, camera table 34, plug 34a (shown in FIG. 1) and receptacle 18a (shown in FIG. 1). The video processor 18 converts the electric signal to a video signal. The monitor 20 displays an endoscopic image.

If the endoscopic image displayed on the monitor 20 is out of focus, the operator may rotate the focus ring 102 (shown in FIG. 34) to adjust the position of the focus. As the focus ring 102 is rotated, the cam mechanism (not shown) moves the movable part 104 moves back or forth along the optical axis of the optical system 74. As the movable part 104 so moves, a cam mechanism (not shown) moves the lens frame 72 back or forth along the optical axis. The focus adjustment is thereby accomplished in the endoscope.

How the switch device 52 operates to make the endoscopic image brighter on the monitor 20.

As shown in FIG. 35A, the distance Lₐ between the magnet 314 and the first magnetic member 320a is shorter than the distance L_{b} between the magnet 314 and the second magnetic member 320b until the input unit 112 is pushed with respect to the outer shell 44 (or while the input unit 112 remains at the first position). The magnetic fluxes are therefore oriented in the first direction. Thus, the density of magnetic fluxes is higher in the first direction than in the second direction. Hence, the magnetic sensor 322 cannot detect magnetic forces and remains off.

Thereafter, the operator may push the input unit 112 with respect to the outer shell 44 as is illustrated in FIG. 35B. Then, the input unit 112 is deformed, decreasing the distance L_{b} between the magnet 314 and the second magnetic member 320b. When the distance L_{b} between the magnet 314 and the second magnetic member 320b becomes shorter than the distance Lₐ between the magnet 314 and the first magnetic member 320a, the direction in which the magnetic fluxes are oriented changes to the second direction. Thus, the density of the magnetic fluxes is higher in the second direction than in the first direction. Hence, the magnetic sensor 322 provided between the magnet 314 and the second magnetic member 320b detects the magnetic force and is turned on.

The magnetic sensor 322 generates a signal when it is turned on. The signal is supplied from the magnetic sensor 322 to the video processor 18 via the rigid board 324, second connector 92b, first connector 92b, signal-transmitting circuit 80, hermetic connector 66, camera table 34, plug 34a and receptacle 18a. The video processor 18 processes the signal, whereby the endoscopic image displayed on the monitor 20 becomes brighter by a prescribed value.

When the operator stops pushing the input unit 112 with a finger, the input unit 112 returns to the initial position (first position) by virtue of the elastic force it has. Then, the magnetic sensor 322 can no longer detect magnetic forces equal to or greater than a predetermined magnitude. The sensor 322 is therefore turned off. The endoscopic image maintains the brightness. When the input unit 122 is pushed for another time, the brightness of the endoscopic image is further increased by the prescribed value, in the same way as described above.

The input unit 112 may be repeatedly operated to increase the brightness of the image. When the image reaches the maximum brightness, the input unit 112 may be pushed. Then, the magnetic sensor 322 is turned on. At this point, the brightness of the endoscopic image decreases to the lower limit of a present brightness range. Thereafter, the input unit 112 may be repeatedly pushed. Every time the input unit 122 is pushed, the endoscopic image becomes brighter by a prescribed value.

Alternatively, the operating mode of the magnetic sensor 322 may be set as follows. When the input unit 112 is pushed after the endoscopic image has attained a predetermined brightness by repeatedly pushing the unit 112, the input switch 122 is depressed and turned on. At this time, the endoscopic image becomes darker by the prescribed value. Thereafter, as the input unit 112 is repeatedly pushed, the endoscopic image becomes gradually darker, each time by the prescribed value. If the input unit 112 is repeatedly pushed after the endoscopic image has become the darkest, the endoscopic image will become brighter, each time by the prescribed value.

### (Advantage of The Embodiment)

In view of the foregoing, the following can be said of the embodiment.

The direction in which the magnetic fluxes are oriented, mainly toward the first magnetic member 320a or the second magnetic member 320b, can be changed in accordance with how the input unit 112 is operated with respect to the outer shell 44. The magnetic sensor 322 can therefore be turned on and off. Ultimately, the switch device 52 can be on and off.

The stroke of the input unit 112 can be small. Hence, the operability of the switch device 52 can be enhanced.

The magnet 314 may be of such type as overcomes the difference in operating characteristic between magnetic sensors 322. In this case, there is no need to select and use a magnetic sensor. The endoscope system 10 can be provided at a low price.

The video processor 18 shown in FIG. 1 can be remote-controlled to perform various functions, such as adjusting the brightness of endoscopic images and freezing the images, in accordance with the output of the magnetic sensor 322. The number of switch devices 52 is not limited to one (as shown in FIGS. 35A and 35B). A plurality of switch devices may be used.

As has been described, the switch device 52 is integrally formed with the image-pickup device 14 for use in endoscopes. Nonetheless, its structure may be applied to a remote-switch unit, a surgical hand-piece unit or the like, which is a unit independent of the image-pickup device 14. Further, the endoscope 12 and the image-pickup device 14 may be combined, forming a single unit. Such a single unit may be used as an electronic endoscope that has an insertion section 24 and a solid-state imaging element 78 secured to the distal end of the insertion section 24.

### [Twenty-First Embodiment]

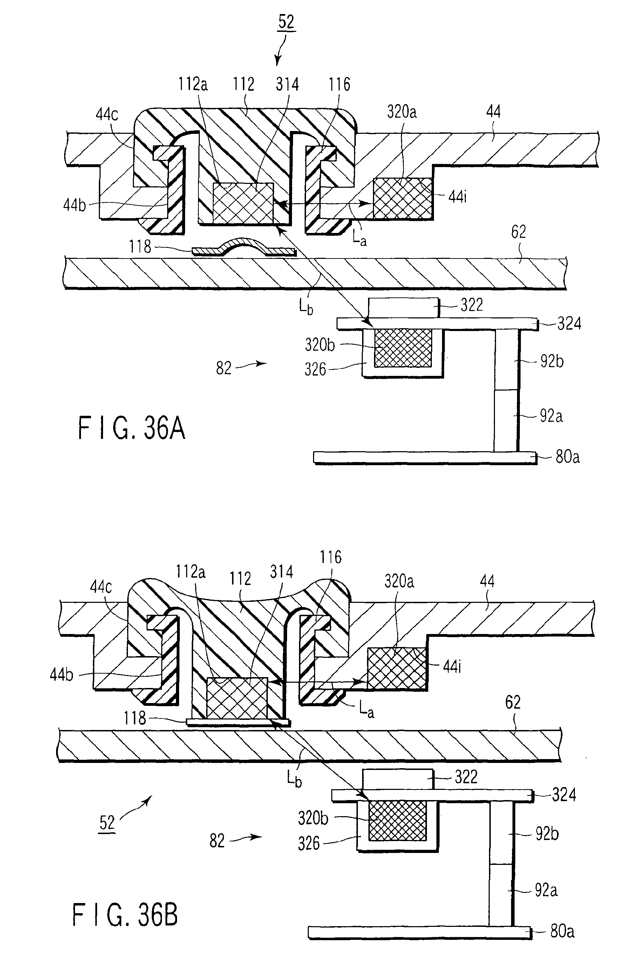

The twenty-first embodiment will be described with reference to FIGS. 36A and 36B. The embodiment is a modification of the twentieth embodiment. The components which are identical to those of the twentieth embodiment are designated by the same reference numbers and will not be described in detail.

### (Configuration)

As shown in 36A and 36B, the twenty-first embodiment differs from the twentieth embodiment in the arrangement of the switch unit 82. In the embodiment, the switch unit 82 is arranged, facing the first magnetic member 320a.

The magnet 314 and the first magnetic member 320a are spaced apart by a distance Lₐ. The magnet 314 and the second magnetic member 320b are spaced apart by a distance L_{b}. The distance L_{b} is longer than the distance Lₐ while the magnet 314 stays at the first position (see FIG. 36A). The distance Lₐ is longer than the distance L_{b} while the magnet 314 stays at the second position (see FIG. 36B). Thus, while the magnet 314 stays at the first position, magnetic fluxes converge, oriented (defining density) from the magnet 314 toward the first magnetic member 320a (in the first direction). On the other hand, while the input unit 112 stays at the second position, the magnetic fluxes converge, oriented (defining density) from the magnet 314 toward the second magnetic member 320b (in the second direction).

### (Operation)

Assume that the switch device 52 has the function of increasing the brightness of endoscopic images. It will be explained, based on this assumption, how the switch device 52 operates to increase the brightness of the endoscopic image displayed on the monitor 20.

As shown in FIG. 36A, the distance Lₐ between the magnet 314 and the first magnetic member 320a is shorter than the distance L_{b} between the magnet 314 and the second magnetic member 320b until the input unit 112 is pushed. The magnetic fluxes are therefore oriented in the first direction. Hence, the magnetic sensor 322 cannot detect magnetic forces and remains off.

Thereafter, the operator may push the input unit 112 with a finger as is illustrated in FIG. 36B. Then, the input unit 112 is deformed, increasing the distance Lₐ between the magnet 314 and the first magnetic member 320a. When the distance Lₐ between the magnet 314 and the first magnetic member 320a becomes longer than the distance L_{b} between the magnet 314 and the second magnetic member 320b, the direction in which the magnetic fluxes are oriented changes, from the first direction to the second direction. Hence, the magnetic sensor 322 provided between the magnet 314 and the second magnetic member 320b detects the magnetic force and is turned on.

The magnetic sensor 322 generates a signal when it is turned on. The signal is supplied from the magnetic sensor 322 to the video processor 18 via the rigid board 324, second connector 92b, first connector 92b, signal-transmitting circuit 80, hermetic connector 66 shown in FIG. 34, camera table 34, plug 34a shown in FIG. 1 and receptacle 18a. The video processor 18 processes the signal, whereby the endoscopic image displayed on the monitor 20 becomes brighter by a prescribed value.

When the operator stops pushing the input unit 112 with the finger, the input unit 112 returns to the initial position (first position) by virtue of the elastic force it has. Then, the magnetic sensor 322 can no longer detect magnetic forces equal to or greater than a predetermined magnitude. The sensor 322 is therefore turned off. The endoscopic image maintains the brightness. When the input unit 122 is pushed for another time, the brightness of the endoscopic image is further increased by the prescribed value, in the same way as described above.

### (Advantage of the This Embodiment)

The present embodiment can achieve the same advantage as the twentieth embodiment.

### [Twenty-Second Embodiment]

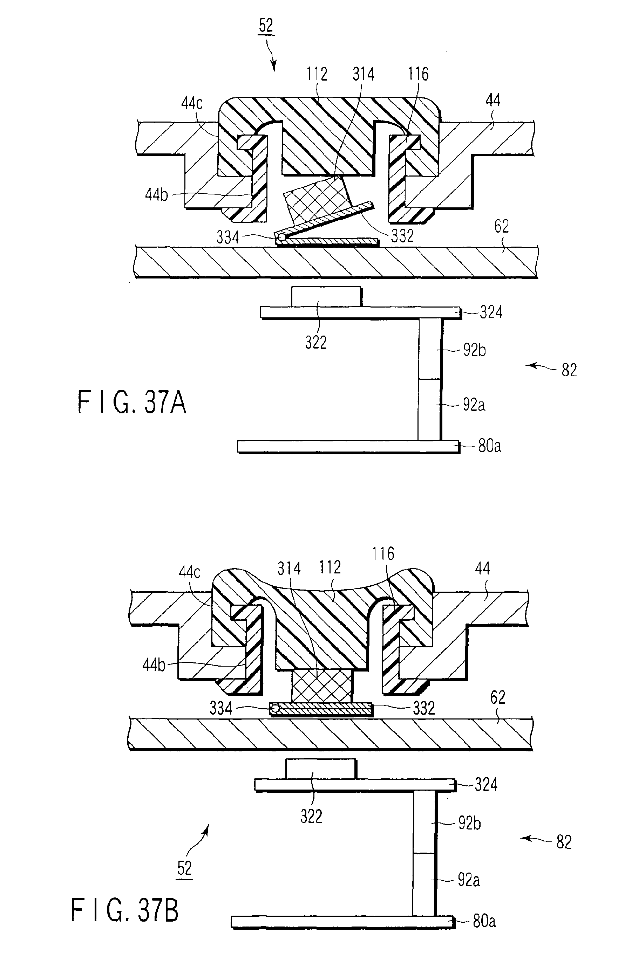

The twenty-second embodiment will be described with reference to FIGS. 37A and 37B. The embodiment is a modification of the twentieth and twenty-first embodiments. The components which are identical to those of the twentieth and twenty-first embodiments are designated by the same reference numbers and will not be described in detail.

### (Configuration)

As shown in FIGS. 37A and 37B, the twenty-second embodiment differs from the twentieth embodiment in the configuration of the switch device 52.

As FIG. 37A shows, the switch device 52 includes a switch unit 82, an input unit 112, a magnet 314, a fastening member 116, and a spring hinge (magnetic-force changing mechanism) 332.

The switch unit 82 includes a magnetic sensor 322, a rigid board 324, a first connector 92a, and a second connector 92b. The second connector 92b is used to transmit an electric signal to the first connector 92a when the magnetic sensor 322 is turned on or off. Neither a second magnetic member 310b nor a magnetic frame 326 is mounted on the rigid board 324.

The input unit 112 does not have the recess 112a (FIGS. 35A to 36B). Nor the leaf spring 118 (FIGS. 35A, 35B, 36B and 36B) is used. Therefore, a space is provided between the input unit 112 and the airtight-unit body 62. In this space, the magnet 314 and the spring hinge 332 are arranged.

The spring hinge 332 is shaped like letter V. A support shaft 334 supports the spring hinge 332, allowing the hinge 332 to open and close. The support shaft 334 is arranged, interesting at right angles to the axis of the input unit 112. One half of the spring hinge 332 is fastened to the airtight-unit body 62 with screws (not shown) or the like. The magnet 314 is secured to the other half of the spring hinge 332, for example, with adhesive. The magnet 314 is positioned to abut on the input unit 112. Therefore, the spring hinge 332 is closed when a pressing force is applied to the other half of the hinge 332 via the magnet 314. The magnet 324 stays at the first position (see FIG. 37A) until the input unit 112 is operated. When the input unit 112 is operated, the magnet 314 moves to the second position (see FIG. 37B). Thus, the magnet 314 is moved in accordance with the motion of the input unit 112.

While the magnet 314 stays at the first position, the magnetic fluxes emanating from the magnet 314 are oriented to the lower-right corner of the drawing (in the first direction). In this case, the density of the magnetic fluxes is low. The magnetic sensor 322 is set not to detect the magnetic force applied from the magnet 314 in this case. While the magnet 314 stays at the second position, the magnetic fluxes are oriented from the magnet 314 to the lower side of the drawing (in the second direction). That is, the fluxes are directed to the magnetic sensor 322. In this case, the density of the magnetic fluxes increases. Then, the magnetic sensor 322 detects the magnetic force applied from the magnet 314. In view of this, the spring hinge 332 is magnetic-force changing means for orientating the magnetic fluxes emanating from the magnet 314, from the first direction to the second direction. The position where the magnetic sensor 322 starts detecting the magnetic force as the magnetic fluxes emanating from the magnet 312 are orientated from the first direction to the second direction can be set as is desired.

### (Operation)

Assume that the switch device 52 has the function of increasing the brightness of endoscopic images. It will be explained, based on this assumption, how the switch device 52 operates to increase the brightness of the endoscopic image displayed on the monitor 20.

As FIG. 37A shows, the magnetic fluxes generated by the magnet 314 are oriented in the first direction because of the bias of the spring hinge 332, until the input unit 112 is pushed (that is, while the magnet 314 stays at the first position). The magnetic sensor 322 cannot detect the magnetic force and remains off.

Thereafter, the operator may push the input unit 112 with a finger as is illustrated in FIG. 37B. Then, the spring hinge 332 is closed in spite of its bias. The direction in which the magnetic fluxes emanating from the magnet 314 gradually changes from the first direction to the second direction. When the magnetic fluxes are oriented almost in the second direction, the magnetic sensor 322 detects the magnetic force and is turned on.

The magnetic sensor 322 generates a signal when it is turned on. The signal is supplied from the magnetic sensor 322 to the video processor 18 via the rigid board 324, second connector 92b, first connector 92b, signal-transmitting circuit 80, hermetic connector 66 shown in FIG. 34, camera table 34, plug 34a shown in FIG. 1 and receptacle 18a. The video processor 18 processes the signal, whereby the endoscopic image displayed on the monitor 20 becomes brighter by a prescribed value.

When the operator stops pushing the input unit 112 with the finger, the input unit 112 returns to the initial position (first position) by virtue of the elastic force it has. The magnet 314 assumes the initial state, because of the bias of the spring hinge 332. Then, the magnetic sensor 322 can no longer detect magnetic forces equal to or greater than a predetermined magnitude. The sensor 322 is therefore turned off. The endoscopic image maintains the brightness. When the input unit 122 is pushed for another time, the brightness of the endoscopic image is further increased by the prescribed value, in the same way as described above.

### (Advantage of The Embodiment)

The present embodiment can achieve the same advantage as the twentieth embodiment.

### [Twenty-Third Embodiment]

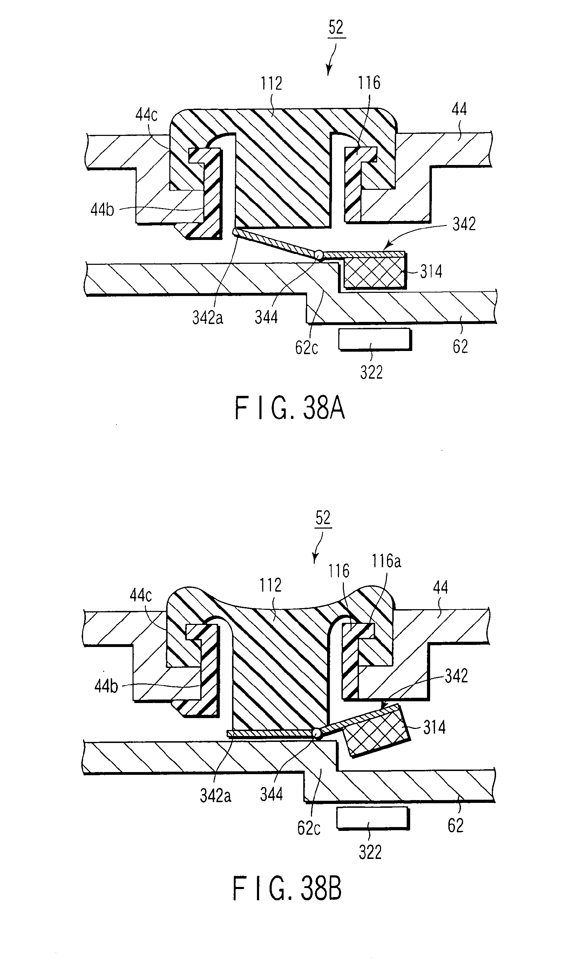

The twenty-third embodiment will be described with reference to FIGS. 38A and 38B. The embodiment is a modification of the twentieth to the twenty-second embodiment. The components which are identical to, or perform the same function as, those of the twentieth to the twenty-second embodiment, are designated by the same reference numbers and will not be described in detail.

### (Configuration)

As FIG. 38A shows, the remote switch device 52 includes a switch unit 82 (magnetic sensor 322), an input unit 112, a magnet 314, a fastening member 116, a plate-like member (magnetic-force changing mechanism) 342, and a support shaft 344. The magnetic sensor 322 shown in FIGS. 38A and 38B is of the same configuration as the switch unit 82 shown in FIGS. 37A and 37B. The components other than the magnetic sensor 322 are not shown for simplicity of the drawing.

The input unit 112 does not have the recess 112a (FIGS. 35A to 36B). Nor the leaf spring 118 (FIGS. 35A to 36B) is used. Therefore, a space is provided between the input unit 112 and the airtight-unit body 62. In this space, the plate-like member 342 is arranged.

The airtight-unit body 62 has a stepped part 62c. Thus, the proximal end of the airtight-unit body 62 has a smaller diameter than the distal end thereof. The stepped part 62c lies, deviated a little from the axis of the input unit 112 toward the proximal end of the airtight-unit body 62.

The plate-like member 342 is shaped like a hinge and has a fixed opening angle. The magnet 314 is secured to, and integrally formed with, one end of the plate-like member 342, by using adhesive or the like. The magnet 314 is located near the airtight-unit body 62, facing the outer circumferential surface of the airtight-unit body 62. The magnet 314 is arranged in the space defined by the stepped part 62c of the airtight-unit body 62.

A pushed part 342a is provided at the other end of the plate-like member 342 and is pushed when the input unit 112 is operated. The plate-like member 342 is supported by a support shaft 344. The support shaft 344 is formed integral with the airtight-unit body 62, by means of adhesion or press-fitting. The support shaft 344 extends at right angles to the axis of the input unit 112.

The input unit 112 can move between the first position (see FIG. 38A) and the second position (see FIG. 38B). The unit 112 stays at the first position until it is operated and moves to the second position when it is operated. While the magnet 314 stays at the first position, the magnetic fluxes remain oriented from the magnet 314 to the lower side of the drawing (in the first direction). That is, they are oriented toward the magnetic sensor 322, which will be described later. When the magnet 314 is moved to the second position, the plate-like member 342 is rotated around the support shaft 344. In this case, the magnetic fluxes are orientated to the lower-right corner of the drawing (in the second direction). In view of this, the plate-like member 342 is magnetic-force changing means for orientating the magnetic fluxes generated by the magnet 314.

The magnetic sensor 322 is arranged in the airtight-unit body 62 that faces the magnet 314 while the magnet 314 stays at the first position. The signal output from the magnetic sensor 322 is processed by the processor 18, whereby the remote switch device 52 is turned on or off. While the magnet 314 remains at the initial position, i.e., the first position (shown in FIG. 38A), the magnetic sensor 322 detects the magnetic force of he magnet 314. In this state, the remote switch device 52 remains off. On the other hand, when the magnet 314 moves (to the second position shown in FIG. 38B) and can no longer detect the magnetic force of the magnet 314 or can detect it but a little, the remote switch device 52 is considered to have been turned on. The position the plate-like member 342 takes when the remote switch device 52 is turned off can be appropriately set.

### (Operation)

Assume that the remote switch device 52 has the function of increasing the brightness of endoscopic images. It will be explained, based on this assumption, how the remote switch device 52 operates to increase the brightness of the endoscopic image displayed on the monitor 20.

As FIG. 38A shows, the magnetic fluxes generated by the magnet 314 are oriented in the first direction until the input unit 112 is pushed (that is, while the magnet 314 stays at the first position). The magnetic sensor 322 therefore detects the magnetic force generated by the magnet 314.

Thereafter, the operator may push the input unit 112 with a finger as is illustrated in FIG. 38B. Then, the input unit 112 is deformed, pushing the pushed part 342a of the plate-like member 342. The plate-like member 342 therefore rotates around the support shaft 344. As a result, the direction in which the magnetic fluxes of the magnet 314 are oriented gradually changes from the first direction to the second direction. At this time, the magnetic sensor 322 detects that the magnetic force generated by the magnet 314 is weak or that the magnetic force has changed. Thus, the remote switch device 52 is turned off, generating a signal. The signal is supplied to the processor 18. The processor 18 processes the signal, whereby the endoscopic image displayed on the monitor 20 becomes brighter by a prescribed value.

When the operator stops pushing the input unit 112 with the finger, the input unit 112 restores its initial state by virtue of the elastic force it has. The magnet 314 assumes the initial state, because of its weight or the like. At this point, the magnet 314 is at the first position with respect to the magnetic sensor 322. Thus, the magnetic sensor 322 detects that the magnetic force from the magnet 314 has become grater than a predetermined magnitude. Hence, the remote switch device 52 is turned off. That is, the output of the remote switch device 52 changes, whereby the brightness of the endoscopic image is maintained. When the input unit 122 is pushed for another time, the brightness of the endoscopic image is further increased by the prescribed value, in the same way as described above.

### (Advantage of The Embodiment)

In the twenty-third embodiment described above, the direction in which the magnetic fluxes are oriented is changed as the input unit 112 is operated. The remote switch device 52 is thereby turned on or off. The stroke of the input unit 112 can be small as the unit 112 is so operated. Hence, the remote switch device 52 can have high operability. Further, the magnet 314 may be of such type as overcomes the difference in operating characteristic between magnetic sensors 322. In this case, there is no need to select and use a magnetic sensor. The remote switch device 52 can therefore be manufactured at a low cost.

### [Twenty-Fourth Embodiment]

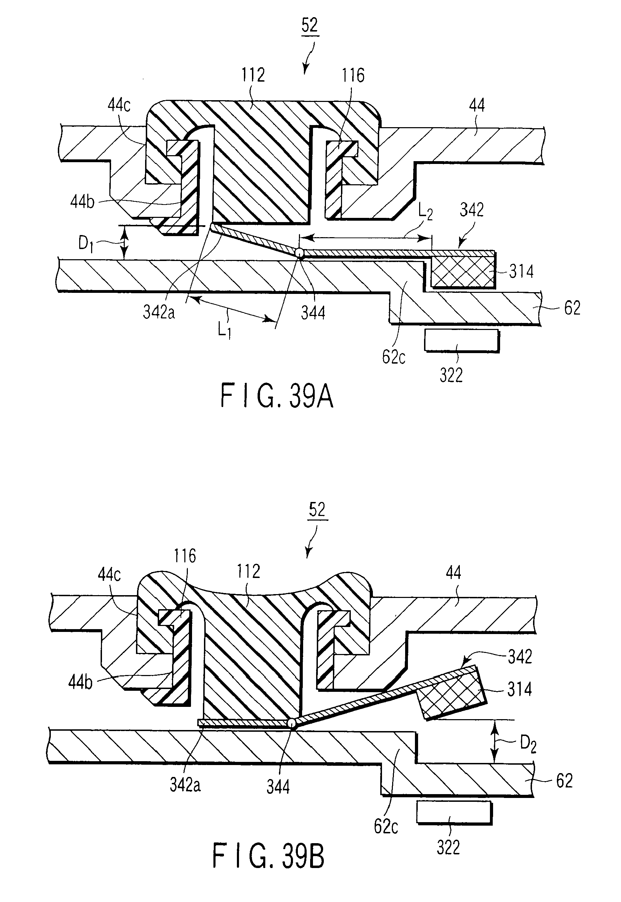

The twenty-fourth embodiment will be described with reference to FIGS. 39A and 39B. The embodiment is a modification of the twenty-third embodiment. The components which are identical to, or perform the same function as, those of the twentieth to the twenty-third embodiment, are designated by the same reference numbers and will not be described in detail.

### (Configuration)

As FIGS. 39A and 39B show, the remote switch device 52 according to the twenty-fourth embodiment differs from the twenty-third embodiment in the shape of the plate-like member 342. The support shaft 344 is spaced apart from the pushed part 342a by distance L₁, and from the magnet 314 by distance L₂. The distance L₂ is longer than the distance L₁. When the input unit 112 is operated, the pushed part 342a moves for distance D₁, and the magnet 314 moves for distance D₂. The distance D₂ is longer than the distance D₁.

### (Operation)

The embodiment operates in the same way as the twenty-third embodiment. Therefore, how it operates will not be explained.

### (Advantage of The Embodiment)

The present embodiment can achieve the same advantage as the twenty-third embodiment. In addition, the magnet 314 can be much moved by pushing the input unit 112 with a small stroke. Thus, not only the direction in which the magnetic fluxes are oriented can be changed, but also the magnet 314 can move away. This decreases the magnetic force fast. The remote switch device 52 can therefore be turned on and off with a smaller stroke than otherwise.

### [Twenty-Fifth Embodiment]

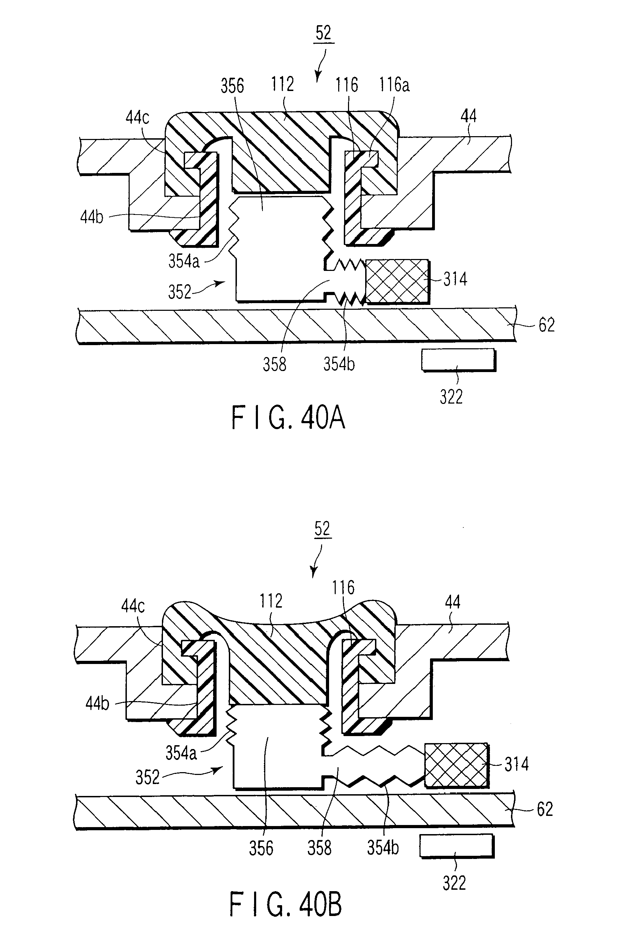

The twenty-fifth embodiment will be described with reference to FIGS. 40A and 40B. The embodiment is a modification of the twenty-second to the twenty-fourth embodiment. The components which are identical to, or perform the same function as, those of the twenty-second to the twenty-fourth embodiment, are designated by the same reference numbers and will not be described in detail.

### (Configuration)

As FIG. 40A shows, the remote switch device 52 includes a switch unit 82 (magnetic sensor 322), an input unit 112, a magnet 314, a fastening member 116, and a deformable member (magnetic-force changing mechanism) 352. The magnetic sensor 322 shown in FIGS. 40A and 40B is of the same configuration as the switch unit 82 shown in FIGS. 37A and 37B. The components other than the magnetic sensor 322 are not shown for simplicity of the drawing.

The input unit 112 does not have the recess 112a (FIGS. 35A, 35B, 36B and 36B). Nor the leaf spring 118 (FIGS. 35A to 36B) is used. Therefore, a space is provided between the input unit 112 and the airtight-unit body 62. In this space, the deformable member 352 is arranged.

The deformable member 352 includes a large-diameter part 356 and a small-diameter part 358, which are coupled in airtight fashion. The large-diameter part 356 has a first bellows 354a, and the small-diameter part 358 has a second bellows 354b. The large-diameter part 356 is arranged in axial alignment with the input unit 112. On the other hand, the small-diameter part 358 is arranged between the recessed part 44c of the outer shell 44 and the airtight-unit body 62. The magnet 314 is integrally formed with the distal end of the small-diameter part 358, by means of adhesion or the like.

The input unit 112 can move between the first position (see FIG. 40A) and the second position (see FIG. 40B). The unit 112 stays at the first position until it is operated and moves to the second position when it is operated. When the unit 112 is operated, it moves to the second position.

The signal output from the magnetic sensor 322 is processed by the processor 18, whereby the remote switch device 52 is turned on or off. While the magnet 314 remains at the initial position, i.e., the first position (shown in FIG. 40A), the magnetic sensor 322 does not detect the magnetic force of the magnet 314. In this state, the remote switch device 52 remains off. On the other hand, at the second position shown in FIG. 40B, the magnetic sensor 322 detects the magnetic force of the magnet 314, and the remote switch device 52 is considered to have been turned on. Thus, the remote switch device 52 is turned on or off, as the magnetic force changes when the magnet 314 moves from the first position to the second position or vice versa.

### (Operation)

Assume that the remote switch device 52 has the function of increasing the brightness of endoscopic images. It will be explained, based on this assumption, how the remote switch device 52 operates to increase the brightness of the endoscopic image displayed on the monitor 20.

As FIG. 40A shows, the magnetic fluxes generated by the magnet 314 are oriented in the first direction until the input unit 112 is pushed (that is, while the magnet 314 stays at the first position). The magnetic sensor 322 therefore detects no magnetic force magnetic forces equal to or greater than a predetermined magnitude. Hence, the remote switch device 52 remains off.

Thereafter, the operator may push the input unit 112 with a finger as is illustrated in FIG. 40B. Then, the input unit 112 is deformed, pushing the deformable member 352. The first bellows 354a of the large-diameter part 356 contracts, whereby air flows from the deformable part 352 into the small-diameter part 358. The second bellows 354b of the small-diameter part 358 therefore expands. Then, the magnet 314 secured to the distal end of the small-diameter part 358 moves, changing the orientation of the magnetic fluxes to the second direction. The magnetic sensor 322 therefore detects the magnetic force applied from the magnet 314. The remote switch device 52 is therefore turned on. As a result, the endoscopic image displayed on the monitor 20 becomes brighter by a prescribed value.

When the operator stops pushing the input unit 112 with the finger, the input unit 112 restores its initial state (that is, moves to the first position) by virtue of the elastic force it has. Air flows from the small-diameter part 358 into the large-diameter part 356. Therefore, the second bellows contracts, whereas the first bellows 354a expands. Hence, the deformable part 352 restores its initial state. At this point, the magnetic sensor 322 can no longer detect magnetic forces equal to or greater than a predetermined magnitude. Hence, the remote switch device 52 turns off. The endoscopic image maintains the brightness. When the input unit 122 is pushed for another time, the brightness of the endoscopic image is further increased by the prescribed value, in the same way as described above.

### (Advantage of The Embodiment)

In the twenty-fifth embodiment thus configured, the remote switch device 52 can be turned on and off, as the input unit 112 is operated to change the direction in which the magnetic fluxes are oriented. Thus, the stroke of the remote switch device 52 can be reduced, enhancing the operability of the remote switch device 52. Since the deformable part 352 includes two parts, i.e., large-diameter part 356 and small-diameter part 358, the remote switch device 52 can be operated with a smaller stroke than otherwise.

Further, the magnet 314 may be of such type as overcomes the difference in operating characteristic between magnetic sensors 322. In this case, there is no need to select and use a magnetic sensor. The remote switch device 52 can therefore be manufactured at a low cost.

### [Twenty-Sixth Embodiment]

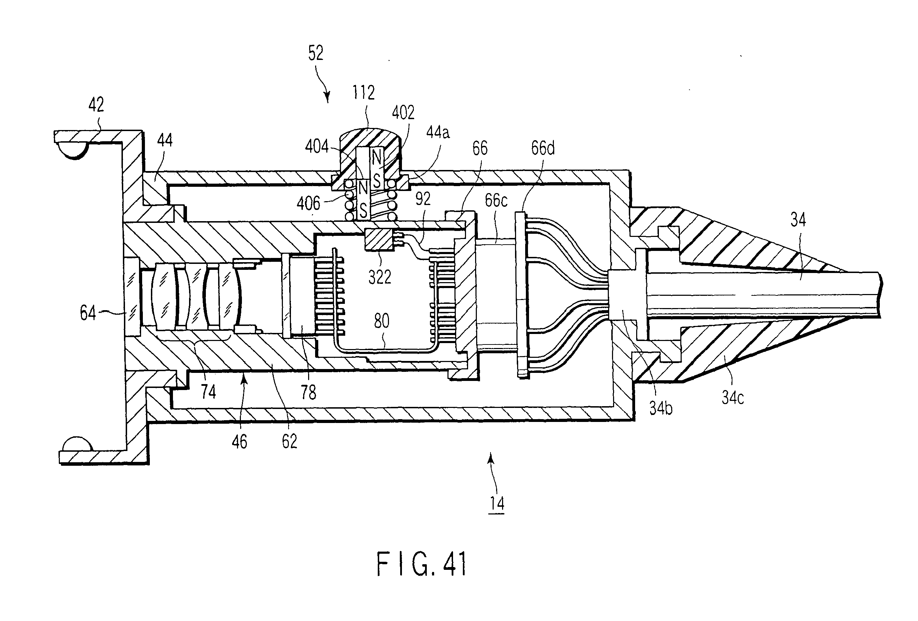

The twenty-sixth embodiment will be described with reference to FIG. 41. The embodiment is a modification of the twentieth embodiment. The components which are identical to, or perform the same function as, those of the twentieth embodiment are designated by the same reference numbers and will not be described in detail.

### (Configuration)

The image-pickup device 14 for use in endoscopes includes a mount 42, an airtight package 46, a camera-head outer shell 44, and a switch device 52. An endoscope 12 can be coupled to the amount 42. The airtight package 46 incorporates observation optics. The switch device 52 can perform remote control.

The structure of the airtight package 46 will be described.

The airtight frame (airtight-unit body) 62 is a hollow cylinder that is made of, for example, stainless steel. The cover glass 64 is secured, by adhesion, to the left end of the airtight frame 62. The optical system (image-pickup lenses) 74 is provided behind the cover glass 64 and secured, by adhesion, to the airtight frame 62.

A processing board 80 is connected, at one end, to a solid-state imaging element 78 by means of soldering. The processing board 80 is configured to process signals supplied from the solid-state imaging element 78. The other end of the processing board 80 is connected to a connector 66 by soldering. A magnetic sensor 322 is secured, by adhesion, to the inner circumferential surface of the airtight frame 62 and located near a magnet 404 that is fixed in the switch device 52. The magnet 404 will be described later. The magnetic sensor 322 is connected by soldering to signal lines 92, which in turn is connected to the contacts of the connector 66. The magnetic sensor 322 is secured to the airtight frame 62 by means of brazing.

Owning to the configuration described above, the air package 46 is completely sealed. Hence, the airtight package 46 protects the optical components and the magnetic sensor 322 against the liquids and steam applied to the switch device 52 from outside.

A cable connector 66c is coupled to the back of the connector 66. More precisely, the connector 66c is secured to a board 66d by means of soldering. The signal lines of the camera cable 34 are soldered to the board 66d.

The camera-head outer shell 44 is fixed by adhesion to the airtight frame 62. A caulking ring 34b and a protection hood 34c are secured by adhesion to the rear end of the camera-head outer shell 44. The caulking ring34b bundles the signal lines of the camera cable 34. The protection hood 34c prevents the buckling of the camera cable 34. The plug 34a is provided on the other end of the camera cable 34. The plug 34a is electrically connected by the video connector 18a to the video processor 18 (see FIG. 1).

The switch device 52 that performs remote control will be described. The switch device 52 includes an input unit 112, a movable magnet (magnetic-force changing mechanism) 402, an immovable magnet (magnetic-force changing mechanism) 404, and a coil spring (magnetic-force changing mechanism) 406. The input unit 112 is made of hard plastic or the like.

The input unit 112 is fitted in a slit 44a cut in the outer shell 44. The input unit 112 is shaped like a cylinder and closed at the upper end (one end). The coil spring 406 is interposed between the outer circumferential surface of the airtight frame 62 and the lower end (other end) of the input unit 112. Thus, the coil spring 406 biases the input unit 112 outwards in the radial direction of the outer shell 44.

The movable magnet 402 is provided in the input unit 112 and adhered to the upper end of the input unit 112, with the coil spring 406 wound around it. The input unit 112 has a space, in which the immovable magnet 404 inserted. Hence, the input unit 112 has a space in which both the movable magnet 402 and the immovable magnet 404 are arranged.

Note that both the movable magnet 402 and the immovable magnet 404 are shaped like a rod. The movable magnet 402 is arranged, with its N pole positioned at the upper end of the input unit 112 and its S pole positioned near the airtight frame 62. The immovable magnet 404 is arranged with its N pole positioned near the upper end of the input unit 112 and its S pole positioned at the airtight frame 62. While the input unit 112 remains in the initial state (or until the input unit 112 is pushed), an attractive force acts between the S pole of the movable magnet 402 and the N pole of the immovable magnet 404. Therefore, the two magnets 402 and 404 define the initial position that the movable magnet 402 has with respect to the immovable magnet 404, i.e., the initial position (first position) that the input unit 112 has with respect to the outer shell 44.

### (Operation)

When the input unit 112 is pushed with respect to the outer shell 44, the movable magnet 402 moves toward the airtight frame 62. The S pole of the movable magnet 402 therefore approaches the S pole of the immovable magnet 404. This induces an abrupt change in the magnetic field between the movable magnet 402 and the immovable magnet 404. As a result, the magnetic field penetrates the airtight frame 62 made of stainless steel, transmitting the change in magnetic field to the magnetic sensor 322.

Thus, the magnetic sensor 322 is turned on when the operator pushes the input unit 112 with respect to the outer shell 44, and is turned off when the operator stops pushing the input unit 112 with a finger, causing the input unit 112 returning to the initial position by virtue of the bias of the spring 406. The output of the magnetic sensor 322 is supplied to the video processor 18 via the signal lines 92, connector 66, cable connector 66c, board 66d and plug 34a. Hence, the input unit 112 performs remote control as it is operated.

### (Advantage of The Embodiment)

In view of the above, the following can be said of the present embodiment.

Since the magnetic sensor 322 is provided in the airtight package 46, the airtight package 46 protects the magnetic sensor 322 against liquids and steam. Therefore, the magnetic sensor 322 hardly has troubles while operating. The electric signal that the switch device 52 is operated to perform remote control can therefore be reliably supplied to the video processor 18.

In the present embodiment, the image-pickup device 14 does not have such a focus-adjusting unit 50 as used in the twentieth embodiment. Nonetheless, it is desirable that the image-pickup device 14 should include a focus-adjusting unit 50 of the same type as used in the twentieth embodiment.

### [Twenty-Seventh Embodiment]

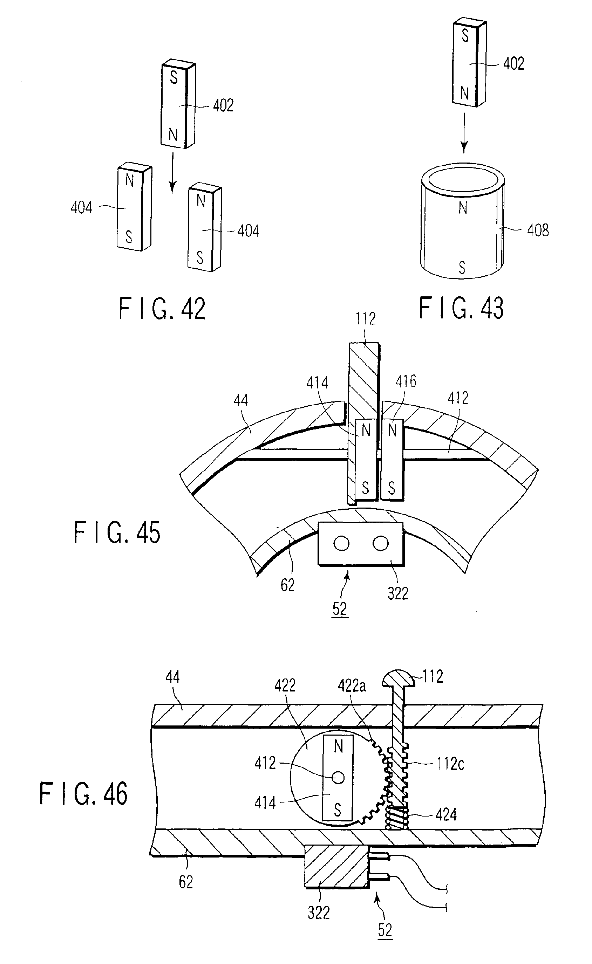

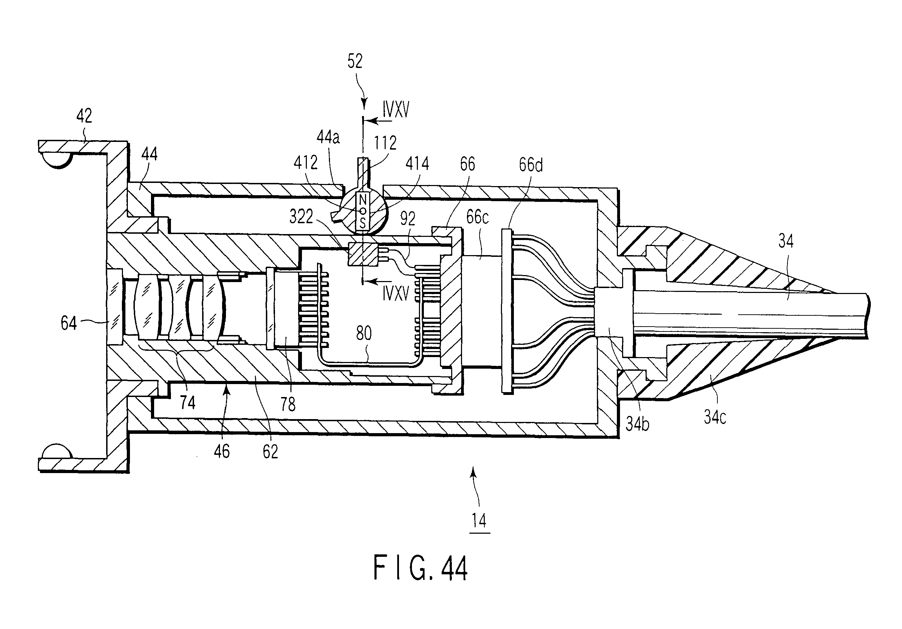

The twenty-seventh embodiment will be described with reference to FIG. 42. The embodiment is a modification of the twenty-sixth embodiment. The components which are identical to, or perform the same function as, those of the twenty-sixth embodiment are designated by the same reference numbers and will not be described in detail.

### (Configuration)

The embodiment uses a pair of immovable magnets 404. The immovable magnets 404 are arranged, with their N poles oriented in the same direction and, hence, their S poles oriented in the same direction. Between the immovable magnets 404 there is provided a space, into which the movable magnet 402 can be inserted and from which the movable magnet 402 can be pulled. The movable magnet 402 is arranged, with its N and S poles oriented in opposite directions to those of either immovable magnet 404.

### (Operation)

The present embodiment operates in the same manner as the twenty-sixth embodiment. Therefore, how it operates will not be explained.

### (Advantage of The Embodiment)

Although the embodiment has one additional immovable magnet 404, it has a simple configuration. Notwithstanding the simple configuration, more abrupt changes can be induced in the magnetic field. This reduces the possibility that the magnetic sensor 322 makes detection errors.

### [Twenty-Eighth Embodiment]

The twenty-eighth embodiment will be described with reference to FIG. 43. The embodiment is a modification of the twenty-sixth and the twenty-seventh embodiment. The components which are identical to, or perform the same function as, those of the twenty-sixth and the twenty-seventh embodiment are designated by the same reference numbers and will not be described in detail.

### (Configuration)

As FIG. 43 shows, one hollow-cylindrical magnet (magnetic-force changing mechanism) 408 is used in place of the immovable magnet 404 (see FIGS. 41 and 42).

### (Operation)

The embodiment operates in the same manner as the twenty-sixth embodiment. Therefore, how it operates will not be explained.

### (Advantage of The Embodiment)

The following can be said of the present embodiment.

The immovable magnet 404 oriented in the opposite direction with respect to the movable magnet 402 is a hollow cylinder. Therefore, a change can be induced in the magnetic field more quickly than in the twenty-seventh embodiment. Hence, the detection ability of the magnetic sensor 322 can be enhanced.

By nature, magnets magnetize one another. It is therefore very troublesome to assemble magnets into the same unit. In other words, the fewer the magnets, the easier it will be to assemble them into a unit. Since one hollow cylindrical magnet 408 is used in place of two magnets, the embodiment can be easier to assemble than the twenty-seventh embodiment.

### [Twenty-Ninth Embodiment]

The twenty-ninth embodiment will be described with reference to FIGS. 44 and 45. The embodiment is a modification of the twenty-sixth embodiment. The components which are identical to, or perform the same function as, those of the twenty-sixth embodiment are designated by the same reference numbers and will not be described in detail.

FIG. 44 shows an airtight package 46 and a remote switch device 52. The airtight package 46 has the illumination optical systems 74 and 78 of an image-pickup device 14 for use in endoscopes. The remote switch device 52 is provided on a camera-head outer shell 44. FIG. 45 is a sectional view taken along line IVXV-IVXV shown in FIG. 44.

### (Configuration)

As FIG. 44 shows, the image-pickup device 14 for use in endoscopes has, as major components, a mount 42, a camera-head outer shell 44, airtight package 46, and a switch device 52. An endoscope 12 can be coupled to the amount 42. The airtight package 46 incorporates observation optics. The switch device 52 can perform remote control.

The structure of the switch device 52 that can perform remote control will be described.

As FIG. 45 shows, the switch device 52 includes a switch plate (input unit) 112, a rotation shaft (magnetic-force changing mechanism) 412, a rotary magnet (magnetic-force changing mechanism) 414, and an immovable magnet (magnetic-force changing mechanism) 416. The switch plate 112 is arranged between the outer shell 44 and the airtight package 46. The rotation shaft 412 penetrates the center of the switch plate 112. The switch plate 112 can rotate around the rotation shaft 412. The rotation shaft 412 extends at right angles to the axis of the image-pickup device 14 for use in endoscopes. As FIG. 45 shows, the rotation shaft 412 has both ends secured to the inner surface of the camera-head outer shell 44, by means of adhesion. The switch plate 112 extends outside, passing through a hole 44b made in the outer shell 44. Therefore, when the switch plate 112 is operated, it rotates around the rotation shaft 412 when it is operated.

As shown in FIGS. 44 and 45, the rotary magnet 414 that is shaped like a rod is fixed to the switch plate 112 by adhesion. As FIG. 44 shows, the immovable magnet 416 is secured to the rotation shaft 412 by adhesion. The rotary magnet 414 and the immovable magnet 416 are spaced apart and extend parallel to each other. The magnets 414 and 416 are arranged, with their S poles and N poles oriented as illustrated in FIG. 45.

### (Operation)

The operator may holds the image-pickup device 14 with hand and rotate the switch plate 112 around the rotation shaft 412, thus tiling the switch plate 112 backwards or forwards. As the switch plate 112 is so moved, the rotary magnet 414 rotates. As a result, the magnetic field between the rotary magnet 414 and the immovable magnet 416 fixed to the rotation shaft 412 changes. The change of the magnetic field is transmitted through the airtight frame 62 made of stainless steel, influencing the magnetic sensor 322 (or changing the magnetic field at the sensor 322). The electric signal generated by the magnetic sensor 322 is supplied to the video processor 18 via the signal lines 92, connectors 66 and 66c, board 66d and camera cable 34. Hence, the preset remote control is turned of or off.

### (Advantage of The Embodiment)

Since the magnetic sensor 322 is provided in the airtight package 46, it hardly has troubles while operating, protected from liquids and steam.

### [Thirtieth Embodiment]

The thirtieth embodiment will be described with reference to FIG. 46. The embodiment is a modification of the twenty-ninth embodiment. The components which are identical to, or perform the same function as, those of the twenty-ninth embodiment are designated by the same reference numbers and will not be described in detail.

### (Configuration)

The embodiment differs from the twenty-ninth embodiment, only in the structures of the switch plate 112 and rotary magnet 414 which are shown in FIGS. 44 and 45.

As FIG. 46 shows, the switch device 52 includes a magnet base 422, a rotation shaft 412, a rotary magnet 414, an immovable magnet 416 (not shown in FIG. 46), a switch pin (input unit) 112, and a coil spring 424.

The magnet base 422 is arranged between the outer shell 44 and the airtight unit 62 of the airtight package 46. The rotation shaft 412 penetrates the center of the magnet base 422. The magnet base 422 can rotate around the rotation shaft 412. The rotation shaft 412 extends at right angles to the axis of the image-pickup device 14 for use in endoscopes. The magnet base 422 is shaped like a disc. A pinion 422a is provided on a part of the outer circumferential surface of the magnet base 422.

The switch pin 112 passes through a hole 44b made in the outer shell 44. Its upper end lies outside the outer shell 44. The switch pin 112 has its lower end supported by the coil spring 424 and can move in its axial direction. The coil spring 424 is secured by adhesion to the outer circumferential surface of the airtight frame 62. The switch pin 112 is always biased outwards in the radial direction of the camera-head outer shell 44. A lack 112c is formed on the switch pin 112 and set in mesh with the pinion 422a provided on the magnet base 422. When the switch pin 112 is rotated, a force is applied from the lack 112c to the pinion 422a provided on the magnet base 422. The magnet base 422 is thereby rotated around the rotation shaft 412.

### (Operation)

When the switch pin 112 is pushed, at the upper end, with respect to the outer shell 44, the magnet base 422 is rotated because the lack 112c on the switch pin 112 is set in mesh with the pinion 422a provided on the magnet base 422. As the magnet base 422 is rotated, the magnetic field between the rotary magnet 414 and the immovable magnet 416 changes in the same manner as in the twenty-ninth embodiment. This change in the magnetic field influences the magnetic sensor 322. As a result, the remote control is turned of or off in accordance the operation of the switch.

### (Advantage of The Embodiment)

The present embodiment can achieve the same advantage as the twenty-ninth embodiment.

The rear ratio between the lack 112c and the pinion 422a may be changed. The magnetic field can then be changed more quickly than in the twenty-ninth embodiment. Further, the detection error can be reduced by setting the gear ration to an appropriate value.

### [Thirty-First Embodiment]

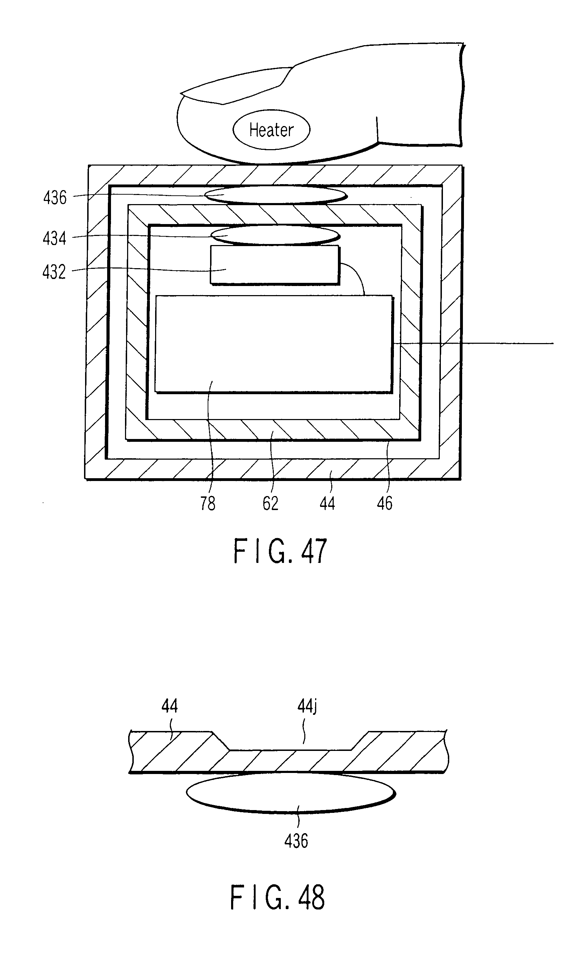

The thirty-first embodiment will be described with reference to FIG. 47. The present embodiment is a modification of the twentieth embodiment. The components which are identical to, or perform the same function as, those of the twentieth embodiment are designated by the same reference numbers and will not be described in detail.

### (Configuration)

As FIG. 47 shows, the switch device 52 used in the image-pickup device 14 for use in endoscopes, according to the present embodiment, includes an outer shell 44, an airtight unit 46, an imaging element 78, a temperature sensor 432, and first and second heat conductors 434 and 436. The conductors 434 and 436 have high thermal conductivity.

The temperature sensor 432 and imaging element 78 are arranged in the airtight frame 62 of the airtight unit 46. The first heat conductor 434 is located between the temperature sensor 432 and the airtight frame 62. The second heat conductor 436 is located between the airtight frame 62 and the outer shell 44. The first and second heat conductors 434 and 436 are opposed to each other, with the airtight frame 62 positioned between them.

### (Operation)

The operator may touch, with finger, that part of the outer shell 44 which is near the first and second heat conductors 434 and 436. Then, heat is transmitted from the operator to the temperature sensor 432 via the outer shell 44, second heat conductor 436, airtight frame 62 and first heat conductor 434. The temperature sensor 432 is turned on, generating a signal. This signal is supplied to the video processor 18 (see FIG. 1) through the signal lines 92. The video processor performs remote control, such as a releasing operation, on an external apparatus (not shown). When the operator stops touching the outer shell 44, heat is no longer applied to the shell 44. The shell 44 is cooled with the atmosphere. As a result, the temperature sensor 432 is turned off.

### (Advantage of The Embodiment)

The following can be said of the present embodiment.

The switch device can be operated, without arranging a special member outside the airtight frame 62, i.e., at a position where steam is applied. Hence, the image-pickup device 14 can have a very high resistance to sterilization using high-pressure steam, without sacrificing its operability.

### [Thirty-Second Embodiment]

The thirty-second embodiment will be described with reference to FIG. 48. The present embodiment is a modification of the thirty-first embodiment. The components which are identical to, or perform the same function as, those of the thirty-first embodiment are designated by the same reference numbers and will not be described in detail.

### (Configuration)

FIG. 48 shows a second heat conductor 436 of the same type used in the thirty-first embodiment and a component provided near the conductor 436. The switch device 52 of the image-pickup device 14 for use in endoscopes, according to the present embodiment, is similar in configuration to that of the thirty-first embodiment. The outer shell 44 arranged near the second heat conductor 436 has a recess 44j.

### (Operation)

As with the thirty-first embodiment, when the operator touches the bottom of the recess 44j made in the outer shell 44 and located near the second heat conductor 436, heat from body temperature (heater) is transmitted from the operator to the temperature sensor 432 via the outer shell 44, second heat conductor 436, airtight frame 62 and first heat conductor 434. The temperature sensor 432 is therefore turned on. The heat is readily transmitted, because that part of the outer shell 44 to which the operator applies heat is thinner than any other part.

### (Advantage of The Embodiment)

The embodiment achieves the same advantage as the thirty-first embodiment. In addition, it can provide a switch that better responds to the operator's touching the outer shell 44.

### [Thirty-Third Embodiment]

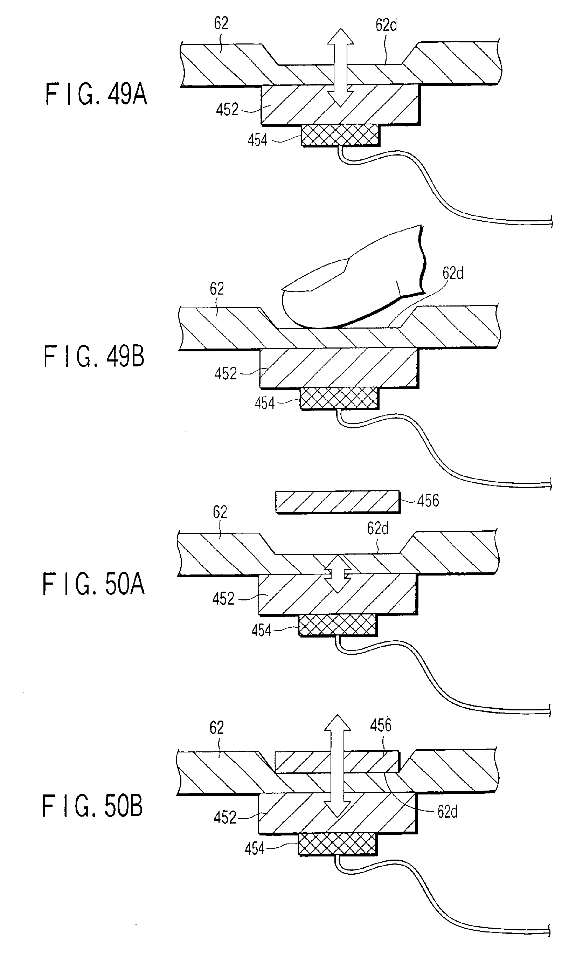

The thirty-third embodiment will be described with reference to FIGS. 49A and 49B. The embodiment is a modification of the thirty-first and the thirty-second embodiment. The components which are identical to, or perform the same function as, those of the thirty-first and the thirty-second embodiment are designated by the same reference numbers and will not be described in detail.

### (Configuration)

As FIG. 49A shows, the switch device 52 used in the image-pickup device 14 for use in endoscopes, according to the present embodiment, includes an airtight frame 62, a vibration source 452, and a vibration sensor (vibration-detecting means) 454. The airtight frame 62 has a thin-wall part 62a. The vibration source 452 is constituted by, for example, a piezoelectric element. The vibration source 452 is interposed between the vibration sensor 454 and the thin-wall part 62d of the airtight frame 62.

### (Operation)

An electric current may be supplied to the vibration source 452. Then, the vibration source 452, the vibration sensor 454, and the thin-wall part 62d of the airtight frame 62 vibrate together, each at its eigenfrequency. The vibration sensor 454 detects its vibration.

The operator may touch the thin-wall part 62d of the airtight frame 62 as shown in FIG. 49B. The force pressing the thin-wall part 62d then suppresses the vibration of the vibration source 452. Therefore, the vibration sensor 454 can no longer detect the vibration of the vibration sensor 454, or the vibration frequency greatly changes. Hence, the vibration sensor 454 detects that the operator has operated the switch device 52. Thus, the switch device 52 remains on while the sensor 454 keeps detecting the vibration, and is turned off when the sensor 454 stops detecting the vibration.

### (Advantage of The Embodiment)

The switch device can be operated, without arranging a special member outside the airtight frame 62, i.e., at a position where steam is applied. Hence, the image-pickup device 14 can have a very high resistance to sterilization using high-pressure steam, without sacrificing its operability.

### [Thirty-Fourth Embodiment]

The thirty-fourth embodiment will be described with reference to FIGS. 50A and 50B. The embodiment is a modification of the thirty-third embodiment. As in the explanation of the thirty-third embodiment, the image-pickup device will not be described and only the switch mechanism and the components peripheral thereto will be described.

### (Configuration)

As FIG. 50A shows, the image-pickup device 14 for use in endoscopes, according to the embodiment, is similar in configuration to that of the thirty-third embodiment. It differs, however, in that a weight 456 is arranged near the thin-wall part 62d of the airtight frame 62 can be fitted in the thin-wall part 62d.

### (Operation)

An electric current may be supplied to the vibration source 452. Then, the vibration source 452, the vibration sensor 454, and the thin-wall part 62d of the airtight frame 62 vibrate together. The vibration source 452 is designed to vibrate at a frequency other than its eigenfrequency so that the vibration amplitude may increase too much due to resonation. When the operator pushes the weight 456, fitting the same into the thin-wall part 62d, the vibration source 452 starts vibrating at its eigenfrequency. Until the weight 456 contacts the airtight frame 62, the vibration amplitude of the vibration source 425 is so small that the vibration sensor 454 cannot detect the vibration. When the weight 456 is fitted in the thin-wall part 62d, the vibration source 452 starts resonating, vibrating at large amplitude. The vibration sensor 454 detects the vibration, generating an on-signal. Thus, it is detected that the operator has pushed the weight 456.

### (Advantage of The Embodiment)

The switch device can be operated, without arranging a special member outside the airtight frame 62, i.e., at a position where steam is applied. Hence, the image-pickup device 14 can have a very high resistance to sterilization using high-pressure steam, without sacrificing its operability.

### [Thirty-Fifth Embodiment]

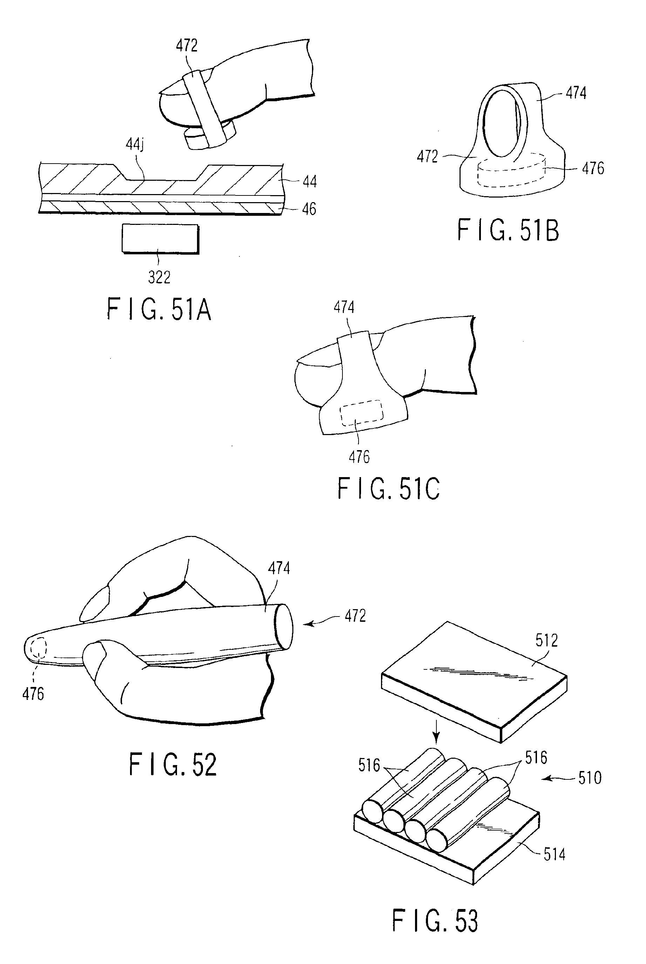

The thirty-fifth embodiment will be described with reference to FIGS. 51A to 51C. The embodiment is a modification of the thirty-first embodiment. The components which are identical to, or perform the same function as, those of the thirty-first embodiment are designated by the same reference numbers and will not be described in detail.

### (Configuration)

As FIG. 51A shows, the switch device 52 used in the image-pickup device 14 for use in endoscopes, according to the present embodiment, has an airtight unit 46, an outer shell 44, a magnetic sensor 322, a recess 44j, and an operation member 472. The airtight unit 46 contains and holds an imaging element (not shown) and optical components (not shown). The recess 44 j is made in the outer surface of the outer shell 44. The operation member 472 may be put on the operator's finger tip.

As shown in FIG. 51B and 51C, the operation member 472 includes an elastic ring-shaped part 474 and a permanent magnet 476. The permanent magnet 476 is attached to the elastic ring-shaped part 474. Using the elastic ring-shaped part 474, the operator may wear the operation member 472 his or her finger tip. The elastic ring-shaped part 474 is as large as the operator's finger is thick. The member 472 is held on the operator's finger, by virtue of the elasticity of the ring-shaped part 474. On the finger tip, the operation member 472 is so positioned that the permanent magnet 476 may be mounted on the finger cushion. It is desired that the bottom of the recess 44j have a printed mark to help the operator to recognize the position of the magnetic sensor 322.

### (Operation)

In preparation for operating the switch, the operator wears the operation member 472 on his or her finger tip. The member 472 is held steady on the finger tip by virtue of the elasticity of the ring-shaped part 474. To operate the switch, the user moves his or her finger tip wearing the operation member 472 toward the recess 44j. When the operation member 472 contacts the outer shell 44, the magnetic force of the permanent magnet 476 acts on the magnetic sensor 322. The magnetic sensor 322 is therefore turned on. As the operation member 472 is moved away from the magnetic sensor 322, the magnetic force acting on the magnetic sensor 322 decreases, whereby the magnetic sensor 322 is turned off.

### (Advantage of The Embodiment)

The switch device can be operated, without arranging a special member outside the airtight frame 62, i.e., at a position where steam is applied. Hence, the image-pickup device 14 can have a very high resistance to sterilization using high-pressure steam, without sacrificing its operability.

### [Thirty-Sixth Embodiment]

The thirty-sixth embodiment will be described with reference to FIG. 52. The present embodiment is a modification of the thirty-fifth embodiment.

### (Configuration)

FIG. 52 shows a rod-shaped operation member 472 that is equivalent to the operation member 472 used in the thirty-fifth embodiment. As FIG. 52 shows, the switch device 52 used in combination with an image-pickup device 14 for use in endoscopes is similar in configuration to that of the thirty-fifth embodiment. Nonetheless, it includes a rod-shaped holding part 474 and a permanent magnet 476. The permanent magnet 476 is fixed to the distal end of the holding part 474.

### (Operation)

As with the thirty-fifth embodiment, the operator may moves the permanent magnet 476 provided at the distal end of the holding part 474 toward the recess 44j made in the outer shell 44 to indicate the position of the magnetic sensor 322. Then, the magnetic force of the permanent magnet 476 acts on the magnetic sensor 322. The magnetic sensor 322 is therefore turned on.

### [Thirty-Seventh Embodiment]

The thirty-seventh embodiment will be described, with reference to FIGS. 53 to 55.

The embodiment uses a magnetic unit (magnetic-force changing mechanism) 510, whose polarity state can be changed by performing an easy operation.

To change the polarity of a magnetic body quickly, the magnetic body must be moved for a larger stroke than the size of the magnetic body. Hence, its polarity can hardly be switched with ease. The polarity of an electromagnet can be indeed switched by changing the direction of the current. However, this involves in electric energy. Here, the configuration of a magnetic unit 510 whose polarity can be quickly switched with a small stroke, thus saving space, without requiring any electric power supply.

### (Configuration)

As FIG. 53 shows, the magnetic unit 510 includes magnetically permeable plates 512 and 514 and at least one permanent magnet 516. The permanent magnet 516 is arranged between the magnetically permeable plates 512 and 514. The magnetically permeable plates 512 and 514 are made of, preferably, magnetic material. The permanent magnet 516 is shaped like a round pillar. The permanent magnet 516 is so positioned that its poles are arranged in a direction perpendicular to its axis. Usually it is desired that a plurality of permanent magnets 516 be used (preferably, a number of small ones). These permanent magnets 516 are arranged so that each has its poles arranged in the same direction as those of any other permanent magnet used.

### (Operation)

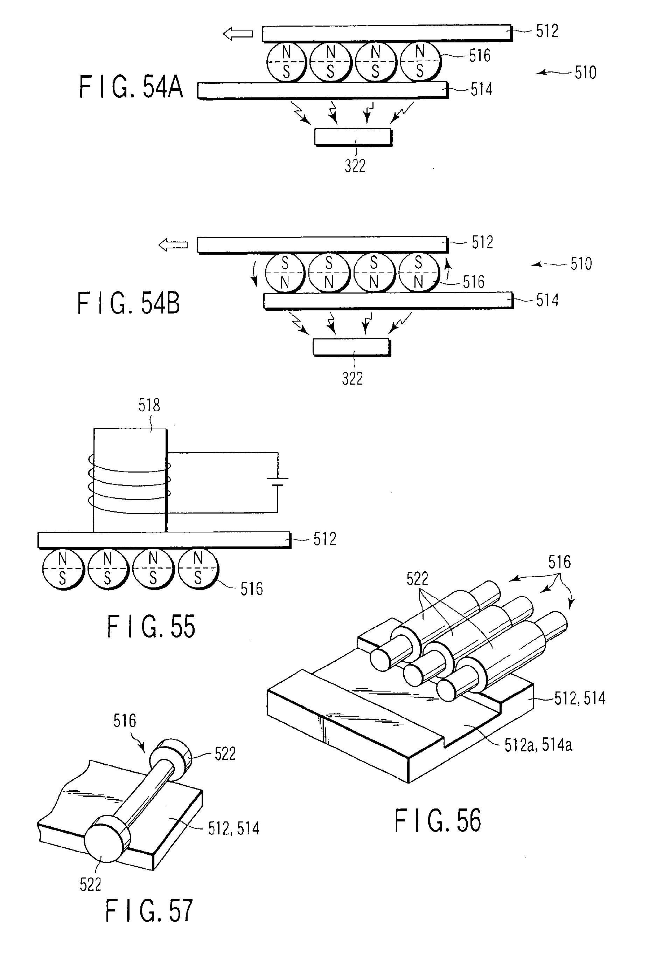

FIGS. 54A and 54B show how one magnetically permeable plate 512 behaves with respect to the other magnetically permeable plate 514 when it is operated.

The permeable plate 512 may be moved with respect to the other permeable plate 514. Then, all permanent magnets 516 interposed between the magnetically permeable plates 512 and 514 roll, whereby the poles of each permanent magnet 516 are changed in position. This changes the polarity of the entire magnetic unit 510.

### (Advantage of The Embodiment)

The polarity state of the magnetic unit 510 can be very drastically switched if the operator moves one magnetically permeable plats 512 only a little. Hence, this change in the polarity can be detected if a magnetic sensor, such as a Hall element 322, is arranged near the other magnetically permeable plate 514. Thus, a switch structure that can detect changes in polarity can be provided.

### (Method of Producing the Magnetic Unit)

As FIG. 55 shows, a method of producing the magnetic unit 510 will be explained.

An electromagnet 518 is attached to one side of one magnetically permeable plate 512. A current is made to flow in the electromagnet 518, magnetically energizing the electromagnet 518. Then, the permanent magnet 516 are arranged on (or attached to) the other side of the magnetically permeable plate 512. Then, the permanent magnets 516 roll to have their poles of either polarity aligned with one anther, because the electromagnet 518 has been magnetically energized. In this state, the other magnetically permeable plate 514 is set into contact with the permanent magnets 516. The permanent magnets 516 are thereby interposed and held between the magnetically permeable plate 512 and 514.

### [Thirty-Eighth Embodiment]

The thirty-eighth embodiment will be described with reference to FIGS. 56 to 59. The embodiment is a modification of the thirty-seventh embodiment. The components which are identical to, or perform the same function as, those of the thirty-seventh embodiment are designated by the same reference numbers and will not be described in detail.

Several methods can be devised, which can increase the operating reliability of the magnetic unit 510 of the type used in the thirty-seventh embodiment. For example, the circumferential surface of each permanent magnet 516 may be roughened, and those sides of the magnetically permeable plates 512 and 514, between which the permanent magnets 516 are interposed and held, may be roughened, too. In this case, the permanent magnets 516 roll more reliably as the magnetically permeable plates 512 is moved:

Otherwise, to enable the magnetically permeable plates 512 and 514 to hold the permanent magnets 516 more steadily, the permanent magnets 516 may not be shaped like a round pillar. Instead, they may have a large-diameter middle part 522 as shown in FIG. 56. If this is the case, both magnetically permeable plates 512 and 514 have grooves 512a and 514a, respectively. In assembling the magnetic unit 510, each permanent magnet 516 is positioned, with its large-diameter part 522 fitted in the grooves 512a and 514a. This prevents the permanent magnets 516 from slipping from the gap between the plates 512 and 514.

Alternatively, each permanent magnet 516 may have two large-diameter parts 522 at both ends as illustrated in FIG. 57, or at one end at least.

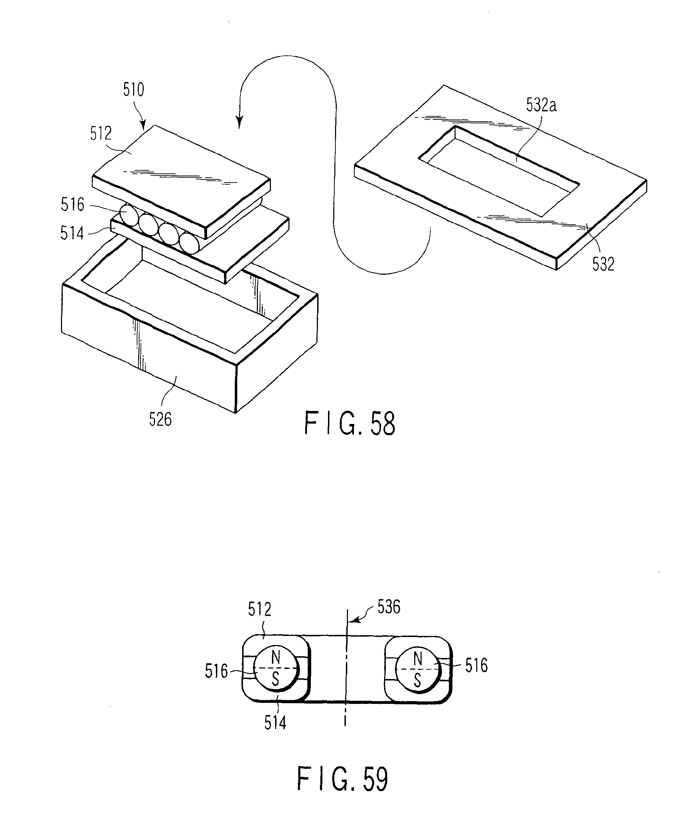

Further, it is desirable to use means for preventing the operator from moving the magnetically permeable plate 512. For example, as shown in FIG. 58, the magnetic unit 510 may be set in a magnetically permeable housing 516. In this case, the magnetically permeable plate 512 and the permanent magnets 516 can move, but within this housing 526 only. This helps to prevent the disintegration of the magnetic unit 510. Moreover, the housing 526 may be closed at top by a cover 532 that has an opening 532a. Then, the magnetically permeable plate 512 can move, but in the opening 532a only. This prevents the disintegration of the magnetic unit 510 even more.

The other magnetically permeable plate 514 may of course be moved, instead of the magnetically permeable plate 512. It suffices if the plates 512 and 514 move relative to each other. For simplicity of description, the magnetically permeable members are plates 512 and 514. Nonetheless, they are not limited to plate-shaped ones, so far as the permanent magnets 516 can roll for a desired distance, while held between the magnetically permeable members 512 and 514.

The permanent magnets 516 are not limited to one shaped like a round pillar. They can have any other shape, only if they can roll in the gap between the magnetically permeable members 512 and 514. For example, they may be shaped like a gear. In this case, two lacks are provided on the magnetically permeable members 512 and 514, respectively. Further, the permanent magnets 516 may be shaped like a ball.

The magnetic unit 510 may be such a bearing-type one as shown in FIG. 59. This unit includes magnetically permeable members 512 and 514 shaped like a disc or ring and permanent magnets 516 shaped like a ball. The permanent magnets 516 are held in a gap between the magnetically permeable members 512 and 514. The magnetically permeable members 512 and 514 are rotated around the axis 536, attaining the same advantage as described above.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A switch mechanism (52) for use in medical apparatuses, **characterized by** comprising:
an airtight unit (46) which is able to retain airtight therein;
a first magnet (114) which is provided outside the airtight unit;
an operation unit (112) on which the first magnet is arranged, which is provided outside the airtight unit and which is able to move the first magnet with respect to the airtight unit;
a second magnet (128) which is provided in the airtight unit and which is able to receive a force from the first magnet in accordance with a position of the first magnet; and
a switch unit (122; 152, 154) which is provided in the airtight unit and which is able to be switched on and off by using a force acting between the first magnet and the second magnet.

2. The switch mechanism (52) according to claim 1,
**characterized in that** the first magnet (114) and the second magnet (128) are arranged to repel each other, and
the switch unit (122; 152, 154) is arranged farther from the first magnet than from the second magnet to be switched by a repulsive force acting between the first magnet and the second magnet.

3. The switch mechanism (52) according to claim 1,
**characterized in that** the first magnet (114) and the second magnet (128) are arranged to attract each other, and
the switch unit (122; 152, 154) is arranged between the first magnet and the second magnet to be switched by an attractive force acting between the first magnet and the second magnet.

4. The switch mechanism (52) according to claim 1,
**characterized in that** the operation unit (112) comprises a return mechanism (112b, 142) which automatically moves the operation unit from a position that the operation unit takes after operated, to a position that the operation unit takes before operated.

5. The switch mechanism (52) according to claim 4,
**characterized in that** the return mechanism (112b) uses an elastic force of the operation unit to automatically return the operation unit to the position that the operation unit takes before operated, thereby moving the first magnet and the second magnet away from each other.

6. The switch mechanism (52) according to claim 4,
**characterized in that** the return mechanism (142) comprises an elastic member which biases the operation unit (112) to move away from the switch unit (122; 152, 154).

7. A switch mechanism (52) for use in medical apparatuses, **characterized by** comprising:
an airtight unit (46) which is able to retain airtight therein;
a magnetic switch (168, 322) which is provided in the airtight unit and which is able to detect changes in a magnetic force;
a magnetic-force generating unit (114; 314; 414) which is provided outside the airtight unit; and
a magnetic-force changing mechanism (112; 160, 162; 20; 320a, 320b; 332; 342; 352; 402, 404, 406, 408; 412, 414, 416; 510) which is able to change a magnetic force acting between a magnet and the magnetic switch.

8. The switch mechanism (52) according to claim 7,
**characterized in that** the magnetic-force changing mechanism (210) comprises an operation unit which moves outside the airtight unit (46), the operation unit being arranged between the magnet (114) and the magnetic switch (168).

9. The switch mechanism (52) according to claim 8,
**characterized in that** the magnetic-force changing mechanism (210) comprises a return mechanism (216) which automatically moves the operation unit from a position that the operation unit takes after operated, to a position that the operation unit takes before operated.

10. The switch mechanism (52) according to claim 7,
**characterized in that** the magnetic-force changing mechanism (112; 160; 210) comprises an operation unit (112; 160; 210) which is configured to move the magnet, the operation unit being arranged the magnet (114; 314; 402, 404; 414, 416).

11. The switch mechanism (52) according to claim 10,
**characterized in that** the operation unit (112; 160; 210) comprises a return mechanism (112b; 164; 216) which automatically moves the operation unit from a position that the operation unit takes after operated, to a position that the operation unit takes before operated.

12. The switch mechanism (52) according to claim 7,
**characterized in that** the magnetic-force changing mechanism (320a, 320b) comprises a first magnetic member (320a) provided outside the airtight unit (46) and a second magnetic member (320b) provided in the magnetic switch (322).

13. A switch mechanism (52) for use in medical apparatuses, **characterized by** comprising:
an airtight unit (46) which is able to retain airtight therein;
a first magnet (114) which is provided outside the airtight unit;
an operation unit (182; 210a; 254; 62b) on which the first magnet is arranged, which is provided outside the airtight unit and which is able to move the first magnet with respect to the airtight unit;
a second magnet (128) which is provided in the airtight unit and which is able to receive a force from the first magnet in accordance with a position of the first magnet; and
a detection switch unit (184) which is provided in the airtight unit, which has a light-emitting part (184a; 224) being able to emit light and a light-receiving part (184b; 222; 222a, 222b) being able to receive light emitted from the light-emitting part, and which is able to detect a change in an amount of light received by the light-receiving part, in accordance with the position of the second magnet.

14. The switch mechanism (52) according to claim 13,
**characterized in that** the second magnet (128) is arranged between the light-emitting part (184a) and the light-receiving part (184b) and is able to move between a position where the second magnet cuts off the light coming from the light-emitting part and a second position where the second magnet allows passage of the light coming from the light-emitting part.

15. The switch mechanism (52) according to claim 13, **characterized by** further comprising:
a first board (80a) which is secured to the airtight unit; and
a second board (124) which is able to move together with the second magnet,
wherein the light-emitting part (224) is provided on the first board or the second board, and
the light-receiving part (222, 222a, 222b) stays on the second board while the light-emitting part remains on the first board and stays on the first board while the light-emitting part remains on the second board.

16. An image-pickup device (14) for use in endoscopes, **characterized by** comprising:
a switch mechanism (52) for use in medical apparatuses, as described in claim 1;
an outer shell (44) which is arranged outside the air-tight unit of the switch mechanism;
an observation optical system (74, 78) which is provided in the airtight unit; and
a cover glass (64) which is arranged at the airtight unit and which is optically connected to the observation optical system provided in the airtight unit.

17. An image-pickup device (14) for use in endoscopes, **characterized by** comprising:
a switch mechanism (52) for use in medical apparatuses, as described in claim 7;
an outer shell (44) which is arranged outside the air-tight unit of the switch mechanism;
an observation optical system (74, 78) which is provided in the airtight unit; and
a cover glass (64) which is arranged at the airtight unit and which is optically connected to the observation optical system provided in the airtight unit.
